# EUROPEAN PATENT SPECIFICATION

(11) **EP 3 449 019 B1**
(45) Date of publication and mention of the grant of the patent: **23.08.2023**
(21) Application number: 17790615.3
(22) Date of filing: 29.04.2017
(51) Int. Cl.: C12Q 1/6827, C12Q 1/6858

(54) **MULTIPLEXED OPTIMIZED MISMATCH AMPLIFICATION (MOMA)-TARGET NUMBER**
ZIELNUMMER ZUR GEMULTIPLEXTEN OPTIMIERTEN FEHLANPASSUNGSVERSTÄRKUNG (MOMA)
NOMBRE DE CIBLES PAR AMPLIFICATION DE MÉSAPPARIEMENT (MOMA) OPTIMISÉE MULTIPLEXÉE (MOA)

(30) Priority: 29.04.2016 US 201662330053 P
(43) Date of publication of application: 06.03.2019
(73) Proprietor: The Medical College of Wisconsin, Inc., Milwaukee, WI 53226 (US)
(72) Inventor: MITCHELL, Aoy, Tomita, Elm Grove, WI 53122 (US); STAMM, Karl, Wauwatosa, WI 53213 (US)
(74) Representative: Dolphin, Kirsty Mairi
(86) International application number: PCT/US2017/030293
(87) International publication number: WO 2017/190106

(56) References cited:
- WO-A1-2004/078999
- WO-A1-2014/143989
- WO-A1-2016/176662
- WO-A2-2015/178978
- US-A1- 2015 056 617
- US-A1- 2015 086 477
- ERIN P. PRICE ET AL: "Cost-effective interrogation of single nucleotide polymorphisms using the mismatch amplification mutation assay and capillary electrophoresis", ELECTROPHORESIS, vol. 31, no. 23-24, December 2010 (2010-12), pages 3881-3888, XP055617581, ISSN: 0173-0835, DOI: 10.1002/elps.201000379
- CHEN ZHANG ET AL: "A Novel Multiplex Tetra-Primer ARMS-PCR for the Simultaneous Genotyping of Six Single Nucleotide Polymorphisms Associated with Female Cancers", PLOS ONE, vol. 8, no. 4, 17 April 2013 (2013-04-17), page e62126, XP055383308, DOI: 10.1371/journal.pone.0062126
- MAK C M ET AL: "Rapid diagnosis of Wilson disease by a 28-mutation panel: Real-time amplification refractory mutation system in diagnosing acute Wilsonian liver failure", CLINICA CHIMICA ACTA, ELSEVIER BV, AMSTERDAM, NL, vol. 398, no. 1-2, 8 August 2008 (2008-08-08), pages 39-42, XP025573813, ISSN: 0009-8981, DOI: 10.1016/J.CCA.2008.08.002 [retrieved on 2008-08-08]
- BURGSTALLER JOERG P ET AL: "Mitochondrial DNA heteroplasmy in ovine fetuses and sheep cloned by somatic cell nuclear transfer", BMC DEVELOPMENTAL BIOLOGY, BIOMED CENTRAL LTD., LONDON, GB, vol. 7, no. 1, 21 December 2007 (2007-12-21), page 141, XP021032212, ISSN: 1471-213X
- STEINBORN R ET AL: "Coexistence of Bos taurus and B. indicus mitochondrial DANs in nuclear transfer-derived somatic cattle clones", GENETICS, GENETICS SOCIETY OF AMERICA, AUSTIN, TX, US, vol. 162, no. 2, October 2002 (2002-10), pages 823-829, XP002969934, ISSN: 0016-6731
- ANDRÉ HOERNING ET AL: "Quantitative real-time ARMS-qPCR for mitochondrial DNA enables accurate detection of microchimerism in renal transplant recipients", PEDIATRIC TRANSPLANTATION, vol. 15, no. 8, 4 December 2011 (2011-12-04), pages 809-818, XP055305029, DK ISSN: 1397-3142, DOI: 10.1111/j.1399-3046.2011.01581.x
- TAIRA C ET AL: "Quantitative monitoring of single nucleotide mutations by allele-specific quantitative PCR can be used for the assessment of minimal residual disease in patients with hematological malignancies throughout their clinical course", CLINICA CHIMICA ACTA, ELSEVIER BV, AMSTERDAM, NL, vol. 412, no. 1-2, 14 January 2011 (2011-01-14), pages 53-58, XP027508889, ISSN: 0009-8981, DOI: 10.1016/J.CCA.2010.09.011 [retrieved on 2010-11-19]
- HIDESTRAND MATS ET AL: "Highly Sensitive Noninvasive Cardiac Transplant Rejection Monitoring Using Targeted Quantification of Donor-Specific Cell-Free Deoxyribonucleic Acid", JOURNAL OF THE AMERICAN COLLEGE OF CARDIOLOGY, ELSEVIER, NEW YORK, NY, US, vol. 63, no. 12, 16 October 2013 (2013-10-16), pages 1224-1226, XP028831507, ISSN: 0735-1097, DOI: 10.1016/J.JACC.2013.09.029
- ANDREW B. SPARKS ET AL: "Selective analysis of cell-free DNA in maternal blood for evaluation of fetal trisomy", PRENATAL DIAGNOSIS, vol. 32, no. 1, 2012, pages 3-9, XP055024132, ISSN: 0197-3851, DOI: 10.1002/pd.2922
- MANAGE ET AL.: 'Genotyping Single Nucleotide Polymorphisms in Human Genomic DNA with an Automated and Self-Contained PCR Cassette' J MOL DIAGN. vol. 16, no. 5, 2014, pages 550 - 557, XP055439200
- BAI ET AL.: 'Detection and Quantification of Heteroplasmic Mutant Mitochondrial DNA by Real- Time Amplification Refractory Mutation System Quantitative PCR Analysis: A Single-Step Approach' MOLECULAR DIAGNOSTICS AND GENETICS vol. 50, no. 6, 2004, pages 996 - 1001, XP055442689
- WILKINS ET AL.: 'IMP PCR primers detect single nucleotide polymorphisms for Anopheles gambiae species identification, Mopti and Savanna rDNA types, and resistance to dieldrin in Anopheles arabiensis' MALARIA JOURNAL vol. 5, 2006, page 125, XP021024130
- NEWTON C R ET AL: "ANALYSIS OF ANY POINT MUTATION IN DNA. THE AMPLIFICATION REFRACTORY MUTATION SYSTEM (ARMS)", NUCLEIC ACIDS RESEARCH, INFORMATION RETRIEVAL LTD, vol. 17, no. 7, 11 April 1989 (1989-04-11) , pages 2503-2516, XP000141596, ISSN: 0305-1048

## Description

### FIELD OF THE INVENTION

This invention relates to methods for assessing an amount of donor-specific cell-free deoxyribonucleic acid (DNA) in a sample from a subject. The methods provided herein can be used to determine risk of a condition, such as transplant rejection. This invention uses multiplexed optimized mismatch amplification (MOMA). US 2015/086477 relates to methods and computer-readable storage media related to cell-free DNA and uses thereof to determine risk of a condition, such as transplant rejection or cancer, in a subject, Burgstaller et al., BMC Developmental Biology, 2007, 7(1): 141, Steinborn et al., Genetics, 2002, 162(2): 823-829, Hoerning et al., Pediatric Transplantation, 2011, 15(8): 809-818, Taira et al., Clinica Chimica Acta, 2011, 412(1-2): 53-58 and Bai et al., Molecular Diagnostics and Genetics, 2004, 50(6): 996-1001 relate to quantitative genotyping methods such as ARMS-PCR.

### SUMMARY OF INVENTION

The present disclosure is based, at least in part on the surprising discovery that multiplexed optimized mismatch amplification (MOMA) can be used to quantify low frequency non-native nucleic acids in samples from a subject. Multiplexed optimized mismatch amplification embraces the design of primers that can include a 3' penultimate mismatch for the amplification of a specific sequence but a double mismatch relative to an alternate sequence. Amplification with such primers can permit the quantitative determination of amounts of non-native nucleic acids in a sample, even where the amount of non-native nucleic acids are, for example, below 1%, or even 0.5%, in a heterogeneous population of nucleic acids.

Provided herein are methods related to such optimized amplification.
In a first aspect, a method of assessing an amount of donor-specific cell-free DNA in a sample from a subject, wherein the subject is a transplant recipient, is provided. The sample comprises donor-specific and subject cell-free DNA, the method comprising for each of 38-40 single nucleotide variant (SNV) targets, performing an amplification-based quantification assay on the sample, or portion thereof, with at least two primer pairs, wherein each primer pair comprises a forward primer and a reverse primer, wherein one of the at least two primer pairs comprises a 3' penultimate mismatch in a primer relative to one allele of the SNV target but a 3' double mismatch relative to another allele of the SNV target and specifically amplifies the one allele of the SNV target, and the another of the at least two primer pairs specifically amplifies the another allele of the SNV target, and obtaining or providing results from the amplification-based quantification assays to determine the amount of donor-specific cell-free DNA in the sample, wherein the donor genotype is not known, and the method further comprises predicting the donor genotype or assessing results based on a prediction of the donor genotype; wherein the results comprise the amount of the donor-specific cell-free DNA in the sample or wherein the method further comprises determining the amount of the donor-specific cell-free DNA in the sample based on the results.

Further embodiments are defined in the appended dependent claims.

### BRIEF DESCRIPTION OF DRAWINGS

The accompanying drawings are not intended to be drawn to scale. The figures are illustrative only and are not required for enablement of the disclosure.
**Fig. 1** provides an exemplary, non-limiting diagram of MOMA primers. In a polymerase chain reaction (PCR) assay, extension of the sequence containing SNV A is expected to occur, resulting in the detection of SNV A, which may be subsequently quantified. Extension of the SNV B, however, is not expected to occur due to the double mismatch.

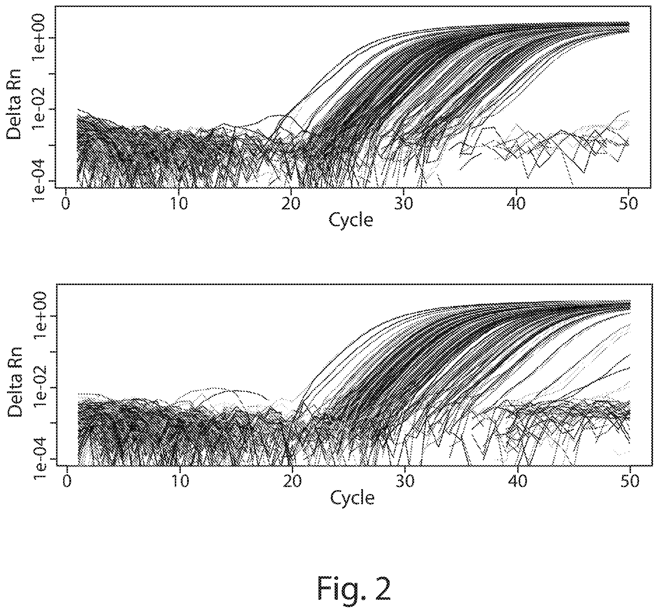

**Fig. 2** provides exemplary amplification traces.
**Fig. 3** shows results from a reconstruction experiment.
**Fig. 4** provides the percent cell-free DNA measured with plasma samples from transplant recipient patients. All data comes from patients who have had biopsies. Dark points denote rejection.
**Fig. 5** provides further data from a method as provided herein on plasma samples. After transplant surgery, the donor percent levels drop off.
**Fig. 6** demonstrates the use of expectation maximization to predict non-native donor genotype when unknown. Dashed line = first iteration, Solid line = second iteration, Final call= 10%.
**Fig. 7** demonstrates the use of expectation maximization to predict non-native donor genotype when unknown. Final call= 5%.
**Fig. 8** provides reconstruction experiment data demonstrating the ability to predict the non-native donor genotype when unknown. Data have been generated with a set of 95 SNV targets.

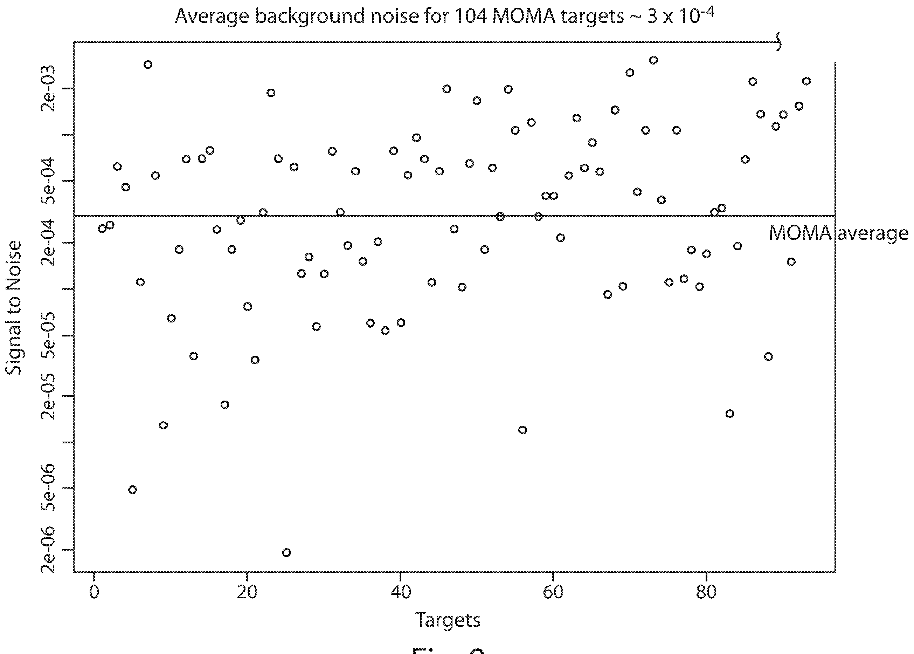

**Fig. 9** provides the average background noise for 104 MOMA targets.

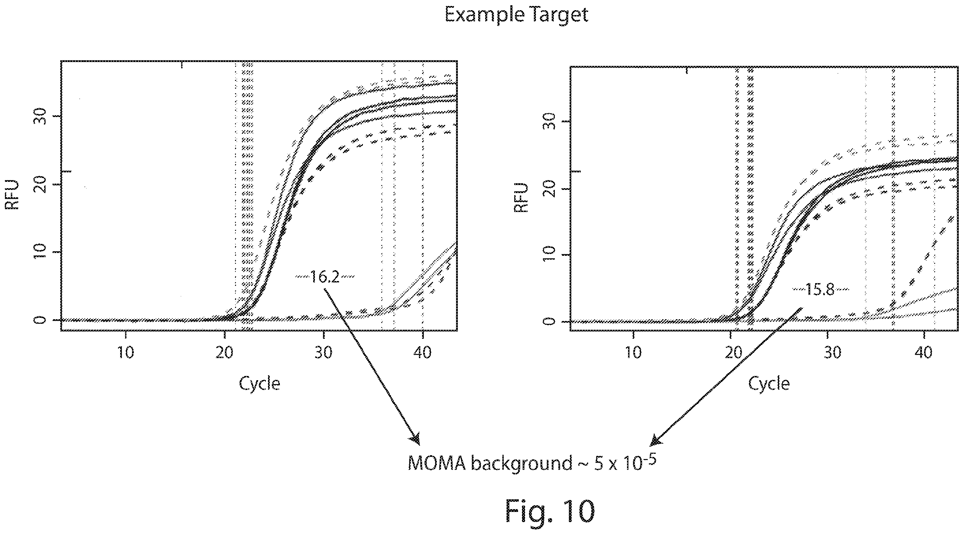

**Fig. 10** provides further examples of the background noise for methods using MOMA.
**Figs. 11** and **12** provide further examples of an EM method (not in accordance with the presently claimed invention).
**Fig. 13** provides an example of a sample run of positive control (PTC).
**Fig. 14** illustrates an example of 17 sample runs of positive control (PTC).
**Fig. 15** shows the donor percent called over different samples when the number of targets are varied.
**Fig. 16** provides results from an exemplary MOMA assay on transplant subjects with (n=12) and without (n=104) graft vasculopathy.
**Fig. 17** illustrates an example of a computer system with which some embodiments may operate.

### DETAILED DESCRIPTION OF THE INVENTION

Aspects of the disclosure relate to methods for the sensitive detection and/or quantification of non-native nucleic acids in a sample. Non-native nucleic acids, such as non-native DNA, may be present in individuals in a variety of situations including following organ transplantation. The disclosure provides techniques to detect, analyze and/or quantify non-native nucleic acids, such as non-native cell-free DNA concentrations, in samples obtained from a subject.

As used herein, "non-native nucleic acids" refers to nucleic acids that are from another source or are mutated versions of a nucleic acid found in a subject (with respect to a specific sequence, such as a wild-type (WT) sequence). "Native nucleic acids", therefore, are nucleic acids that are not from another source and are not mutated versions of a nucleic acid found in a subject (with respect to a specific sequence). In aspects of the invention, the non-native nucleic acid is non-native cell-free DNA. "Cell-free DNA" (or cf-DNA) is DNA that is present outside of a cell, e.g., in the blood, plasma, serum, urine, etc. of a subject. Without wishing to be bound by any particular theory or mechanism, it is believed that cf-DNA is released from cells, e.g., via apoptosis of the cells. An example of non-native nucleic acids are nucleic acids that are from a donor of a transplant in a transplant recipient subject. As used herein, the compositions and methods provided herein can be used to determine an amount of cell-free DNA from a non-native source, such as DNA specific to a donor or donor-specific cell-free DNA (e.g., donor-specific cf-DNA).

Provided herein are methods that can be used to measure nucleic acids with differences in sequence identity. In some embodiments, the difference in sequence identity is a single nucleotide variant (SNV); however, wherever a SNV is referred to herein any difference in sequence identity between native and non-native nucleic acids is intended to also be applicable. Thus, any one of the methods provided herein may be applied to native versus non-native nucleic acids where there is a difference in sequence identity. As used herein, "single nucleotide variant" refers to a nucleic acid sequence within which there is sequence variability at a single nucleotide. In some embodiments, the SNV is a biallelic SNV, meaning that there is one major allele and one minor allele for the SNV. In some embodiments, the SNV may have more than two alleles, such as within a population. In some embodiments, the SNV is a mutant version of a sequence, and the non-native nucleic acid refers to the mutant version, while the native nucleic acid refers to the non-mutated version (such as the wild-type version). Such SNVs, thus, can be mutations that can occur within a subject and which can be associated with a disease or condition. Generally, a "minor allele" refers to an allele that is less frequent, such as in a population, for a locus, while a "major allele" refers to the more frequent allele, such as in a population. The methods and compositions provided herein can quantify nucleic acids of major and minor alleles within a mixture of nucleic acids even when present at low levels, in some embodiments.

The nucleic acid sequence within which there is sequence identity variability, such as a SNV, is generally referred to as a "target". As used herein, a "SNV target" refers to a nucleic acid sequence within which there is sequence variability, such as at a single nucleotide, such as in a population of individuals or as a result of a mutation that can occur in a subject and that can be associated with a disease or condition. The SNV target has more than one allele, and in preferred embodiments, the SNV target is biallelic. In some embodiments of any one of the methods provided herein, the SNV target is a single nucleotide polymorphism (SNP) target. In some of these embodiments, the SNP target is biallelic. It has been discovered that non-native nucleic acids can be quantified even at extremely low levels by performing amplification-based quantitative assays, such as PCR assays with primers specific for SNV targets. In aspects of the invention, the amount of non-native nucleic acids is determined by performing amplification-based quantitative assays, such as quantitative PCR assays, with primers for a plurality of SNV targets.

A "plurality of SNV targets" refers to more than one SNV target where for each target there are at least two alleles. Preferably, in some embodiments, each SNV target is expected to be biallelic and a primer pair specific to each allele of the SNV target is used to specifically amplify nucleic acids of each allele, where amplification occurs if the nucleic acid of the specific allele is present in the sample. In some embodiments, the plurality of SNV targets are a plurality of sequences within a subject that can be mutated and that if so mutated can be indicative of a disease or condition in the subject. As used herein, one allele may be the mutated version of a target sequence and another allele is the non-mutated version of the sequence.

In the method of the invention, the plurality of SNV targets comprises 38-40 informative SNV targets. In the methods provided, the amplification-based quantitative assay, such as quantitative PCR, is performed with primer pairs for at 38-40 informative targets.

In an embodiment of any one of the methods provided herein, primer pairs for SNV targets can be pre-selected based on knowledge that the SNV targets will be informative, such as with knowledge of genotype. In another embodiment of any one of the methods provided herein, however, primer pairs for SNV targets are selected for the likelihood a percentage will be informative. In such embodiments, primer pairs for a greater number of SNV targets are used based on the probability a percentage of which will be informative.

As used herein, "an informative SNV target" is one in which amplification with primers as provided herein occurs, and the results of which are informative. "Informative results" as provided herein are the results that can be used to quantify the level of non-native and/or native nucleic acids in a sample. In some embodiments, informative results exclude results where the native nucleic acids are heterozygous for a specific SNV target as well as "no call" or erroneous call results. From the informative results, allele percentages can be calculated using standard curves, in some embodiments of any one of the methods provided. In some embodiments of any one of the methods provided, the amount of non-native and/or native nucleic acids represents an average across informative results for the non-native and/or native nucleic acids, respectively.

The amount or level, such as ratio or percentage, of non-native nucleic acids may be determined with the quantities of the major and minor alleles as well as the genotype of the native and/or non-native nucleic acids. For example, results where the native nucleic acids are heterozygous for a specific SNV target can be excluded with knowledge of the native genotype. Further, results can also be assessed with knowledge of the non-native genotype. In some embodiments of any one of the methods provided herein, where the genotype of the native nucleic acids is known but the genotype of the non-native nucleic acids is not known, the method may include a step of predicting the likely non-native genotype or determining the non-native genotype by sequencing. Further details of such methods can be found, for example, in PCT Publication No. WO2016/176662,. In some embodiments of any one of the methods provided herein, the alleles can be determined based on prior genotyping of the native nucleic acids of the subject and/or the nucleic acids not native to the subject (e.g., of the recipient and donor, respectively). Methods for genotyping are well known in the art. Such methods include sequencing, such as next generation, hybridization, microarray, other separation technologies or PCR assays. Any one of the methods provided herein can include steps of obtaining such genotypes.

"Obtaining" as used herein refers to any method by which the respective information or materials can be acquired. Thus, the respective information can be acquired by experimental methods, such as to determine the native genotype. Respective materials can be created, designed, etc. with various experimental or laboratory methods, in some embodiments. The respective information or materials can also be acquired by being given or provided with the information, such as in a report, or materials. Materials may be given or provided through commercial means (i.e. by purchasing), in some embodiments.

Reports may be in oral, written (or hard copy) or electronic form, such as in a form that can be visualized or displayed. In some embodiments, the "raw" results for each assay as provided herein are provided in a report, and from this report, further steps can be taken to determine the amount of non-native nucleic acids in the sample. These further steps may include any one or more of the following, selecting informative results, obtaining the native and/or non-native genotype, calculating allele percentages for informative results for the native and non-native nucleic acids, averaging the allele percentages, etc. In other embodiments, the report provides the amount of non-native nucleic acids in the sample. From the amount, in some embodiments, a clinician may assess the need for a treatment for the subject or the need to monitor the amount of the non-native nucleic acids over time. Accordingly, in any one of the methods provided herein, the method can include assessing the amount of non-nucleic acids in the subject at more than one point in time. Such assessing can be performed with any one of the methods or compositions provided herein.

In some embodiments, any one of the methods provided herein may include a step of determining or obtaining the total amount of nucleic acids, such as total cell-free DNA, in one or more samples from the subject. Accordingly, any one or more of the reports provided herein may also include one or more amounts of the total nucleic acids, such as total cell-free DNA, and it is the combination of the amount of non-native nucleic acids and total nucleic acids that is in a report and from which a clinician may assess the need for a treatment for the subject or the need to monitor the subject. In some preferred embodiments of any one of the methods provided herein, the total amount of nucleic acids is determined by a MOMA assay as provided herein and is a measure of native and non-native nucleic acid counts as determined by the MOMA assay, preferably, from informative targets. In some embodiments, the total amount of nucleic acids is determined by any method such as a MOMA assay as provided herein or other assays known to those of ordinary skill in the art but not a MOMA assay as provided herein.

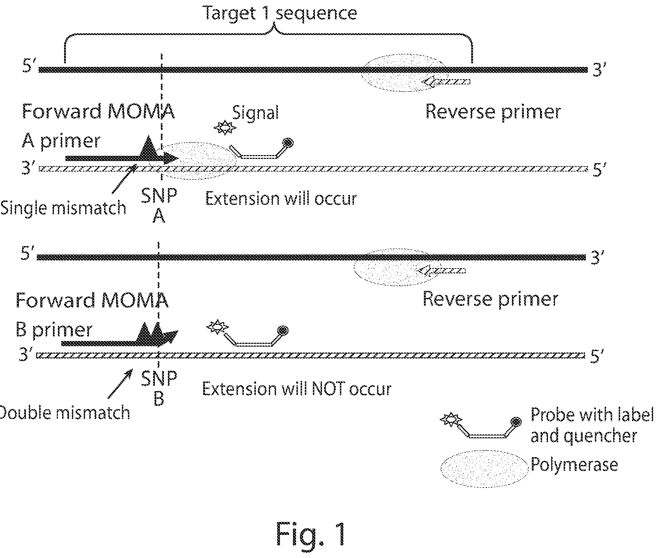

The quantitative assays as provided herein make use of multiplexed optimized mismatch amplification (MOMA). Primers for use in such assays may be obtained, and any one of the methods provided herein can include a step of obtaining one or more primer pairs for performing the quantitative assays. The primers possess unique properties that facilitate their use in quantifying amounts of nucleic acids. For example, a forward primer of a primer pair can be mismatched at a 3' nucleotide (e.g., penultimate 3' nucleotide). In some embodiments of any one of the methods or compositions provided, this mismatch is at a 3' nucleotide but adjacent to the SNV position. In some embodiments of any one of the methods provided, the mismatch positioning of the primer relative to a SNV position is as shown in **Fig. 1****.** Generally, such a forward primer, even with the 3' mismatch, is able to produce an amplification product (in conjunction with a suitable reverse primer) in an amplification reaction, thus allowing for the amplification and resulting detection of a nucleic acid with the respective SNV. If the particular SNV is not present, and there is a double mismatch with respect to the other allele of the SNV target, an amplification product will generally not be produced. In the methods provided herein, for each SNV target a primer pair is obtained whereby specific amplification of each allele can occur without amplification of the other allele(s). "Specific amplification" refers to the amplification of a specific allele of a target without substantial amplification of another nucleic acid or without amplification of another nucleic acid sequence above background or noise. In some embodiments, specific amplification results only in the amplification of the specific allele.

In the methods provided herein, for each SNV target that is biallelic, there are two primer pairs, each specific to one of the two alleles and thus have a single mismatch with respect to the allele it is to amplify and a double mismatch with respect to the allele it is not to amplify (if the nucleic acids of these alleles are present). In some embodiments of any one of the methods provided herein, the mismatch primer is the forward primer. In some embodiments of any one of the methods provided herein, the reverse primer of the two primer pairs for each SNV target is the same.

These concepts can be used in the design of primer pairs for any one of the methods provided herein. It should be appreciated that the forward and reverse primers are designed to bind opposite strands (e.g., a sense strand and an antisense strand) in order to amplify a fragment of a specific locus of the template. The forward and reverse primers of a primer pair may be designed to amplify a nucleic acid fragment of any suitable size to detect the presence of, for example, an allele of a SNV target, according to the disclosure. Any one of the methods provided herein can include one or more steps for obtaining one or more primer pairs as described herein.

It should be appreciated that the primer pairs described herein may be used in a multiplex PCR assay. Accordingly, in some embodiments of any one of the methods provided herein, the primer pairs are designed to be compatible with other primer pairs in a PCR reaction. For example, the primer pairs may be designed to be compatible with at least 2, at least 5, at least 10, at least 15, at least 20, at least 25, at least 30, at least 35, at least 40, etc. other primer pairs in a PCR reaction. As used herein, primer pairs in a PCR reaction are "compatible" if they are capable of amplifying their target in the same PCR reaction. In some embodiments, primer pairs are compatible if the primer pairs are inhibited from amplifying their target DNA by no more than 1%, no more than 2%, no more than 3%, no more than 4%, no more than 5%, no more than 10%, no more than 15%, no more than 20%, no more than 25%, no more than 30%, no more than 35%, no more than 40%, no more than 45%, no more than 50%, or no more than 60% when multiplexed in the same PCR reaction. Primer pairs may not be compatible for a number of reasons including, but not limited to, the formation of primer dimers and binding to off-target sites on a template that may interfere with another primer pair. Accordingly, the primer pairs of the disclosure may be designed to prevent the formation of dimers with other primer pairs or to limit the number of off-target binding sites. Exemplary methods for designing primers for use in a multiplex PCR assay are known in the art or otherwise described herein.

The primer pairs described herein are used in a multiplex PCR assay to quantify an amount of non-native nucleic acids. Accordingly, in some embodiments of any one of the methods provided herein, the primer pairs are designed to detect genomic regions that are diploid, excluding primer pairs that are designed to detect genomic regions that are potentially non-diploid. In some embodiments of any one of the methods provided herein, the primer pairs used in accordance with the disclosure do not detect repeat-masked regions, known copy-number variable regions, or other genomic regions that may be non-diploid.

In some embodiments of any one of the methods provided herein, the amplification-based quantitative assay is any quantitative assay, such as whereby nucleic acids are amplified and the amounts of the nucleic acids can be determined. Such assays include those whereby nucleic acids are amplified with the MOMA primers as described herein and quantified. Such assays include simple amplification and detection, hybridization techniques, separation technologies, such as electrophoresis, next generation sequencing and the like.

In some embodiments of any one of the methods provided herein, the quantitative assays are quantitative PCR assays. Quantitative PCR include real-time PCR, digital PCR, TAQMAN^{™}, etc. In some embodiments of any one of the methods provided herein the PCR is "real-time PCR". Such PCR refers to a PCR reaction where the reaction kinetics can be monitored in the liquid phase while the amplification process is still proceeding. In contrast to conventional PCR, real-time PCR offers the ability to simultaneously detect or quantify in an amplification reaction in real time. Based on the increase of the fluorescence intensity from a specific dye, the concentration of the target can be determined even before the amplification reaches its plateau.

The use of multiple probes can expand the capability of single-probe real-time PCR. Multiplex real-time PCR uses multiple probe-based assays, in which each assay can have a specific probe labeled with a unique fluorescent dye, resulting in different observed colors for each assay. Real-time PCR instruments can discriminate between the fluorescence generated from different dyes. Different probes can be labeled with different dyes that each have unique emission spectra. Spectral signals are collected with discrete optics, passed through a series of filter sets, and collected by an array of detectors. Spectral overlap between dyes may be corrected by using pure dye spectra to deconvolute the experimental data by matrix algebra.

A probe may be useful for methods of the present disclosure, particularly for those methods that include a quantification step. Any one of the methods provided herein can include the use of a probe in the performance of the PCR assay(s), while any one of the compositions of kits provided herein can include one or more probes. Importantly, in some embodiments of any one of the methods provided herein, the probe in one or more or all of the PCR quantification assays is on the same strand as the mismatch primer and not on the opposite strand. It has been found that in so incorporating the probe in a PCR reaction, additional allele-specific discrimination can be provided.

As an example, a TAQMAN^{™} probe is a hydrolysis probe that has a FAM^{™} or VIC^{®} dye label on the 5' end, and minor groove binder (MGB) non-fluorescent quencher (NFQ) on the 3' end. The TAQMAN^{™} probe principle generally relies on the 5'-3' exonuclease activity of Taq^{®} polymerase to cleave the dual-labeled TAQMAN^{™} probe during hybridization to a complementary probe-binding region and fluorophore-based detection. TAQMAN^{™} probes can increase the specificity of detection in quantitative measurements during the exponential stages of a quantitative PCR reaction.

PCR systems generally rely upon the detection and quantitation of fluorescent dyes or reporters, the signal of which increase in direct proportion to the amount of PCR product in a reaction. For example, in the simplest and most economical format, that reporter can be the double-strand DNA-specific dye SYBR^{®} Green (Molecular Probes). SYBR Green is a dye that binds the minor groove of double stranded DNA. When SYBR Green dye binds to a double-stranded DNA, the fluorescence intensity increases. As more double-stranded amplicons are produced, SYBR Green dye signal will increase.

In any one of the methods provided herein, the PCR may be digital PCR. Digital PCR involves partitioning of diluted amplification products into a plurality of discrete test sites such that most of the discrete test sites comprise either zero or one amplification product. The amplification products are then analyzed to provide a representation of the frequency of the selected genomic regions of interest in a sample. Analysis of one amplification product per discrete test site results in a binary "yes-or-no" result for each discrete test site, allowing the selected genomic regions of interest to be quantified and the relative frequency of the selected genomic regions of interest in relation to one another be determined. In certain aspects, in addition to or as an alternative, multiple analyses may be performed using amplification products corresponding to genomic regions from predetermined regions. Results from the analysis of two or more predetermined regions can be used to quantify and determine the relative frequency of the number of amplification products. Using two or more predetermined regions to determine the frequency in a sample reduces a possibility of bias through, e.g., variations in amplification efficiency, which may not be readily apparent through a single detection assay. Methods for quantifying DNA using digital PCR are known in the art and have been previously described, for example in U.S. Patent Publication number US20140242582.

It should be appreciated that the PCR conditions provided herein may be modified or optimized to work in accordance with any one of the methods described herein. Typically, the PCR conditions are based on the enzyme used, the target template, and/or the primers. In some embodiments, one or more components of the PCR reaction is modified or optimized. Non-limiting examples of the components of a PCR reaction that may be optimized include the template DNA, the primers (e.g., forward primers and reverse primers), the deoxynucleotides (dNTPs), the polymerase, the magnesium concentration, the buffer, the probe (e.g., when performing real-time PCR), the buffer, and the reaction volume.

In any of the foregoing embodiments, any DNA polymerase (enzyme that catalyzes polymerization of DNA nucleotides into a DNA strand) may be utilized, including thermostable polymerases. Suitable polymerase enzymes will be known to those skilled in the art, and include E. coli DNA polymerase, Klenow fragment of E. coli DNA polymerase I, T7 DNA polymerase, T4 DNA polymerase, T5 DNA polymerase, Klenow class polymerases, Taq polymerase, Pfu DNA polymerase, Vent polymerase, bacteriophage 29, REDTaq^{™} Genomic DNA polymerase, or sequenase. Exemplary polymerases include, but are not limited to Bacillus stearothermophilus pol I, Thermus aquaticus (Taq) pol I, Pyrccoccus furiosus (Pfu), Pyrococcus woesei (Pwo), Thermus flavus (Tfl), Thermus thermophilus (Tth), Thermus litoris (Tli) and Thermotoga maritime (Tma). These enzymes, modified versions of these enzymes, and combination of enzymes, are commercially available from vendors including Roche, Invitrogen, Qiagen, Stratagene, and Applied Biosystems. Representative enzymes include PHUSION^{®} (New England Biolabs, Ipswich, MA), Hot MasterTaq^{™} (Eppendorf), PHUSION^{®} Mpx (Finnzymes), PyroStart^{®} (Fermentas), KOD (EMD Biosciences), Z-Taq (TAKARA), and CS3AC/LA (KlenTaq, University City, MO).

Salts and buffers include those familiar to those skilled in the art, including those comprising MgCl₂, and Tris-HCl and KCl, respectively. Typically, 1.5-2.0nM of magnesium is optimal for Taq DNA polymerase, however, the optimal magnesium concentration may depend on template, buffer, DNA and dNTPs as each has the potential to chelate magnesium. If the concentration of magnesium [Mg²⁺] is too low, a PCR product may not form. If the concentration of magnesium [Mg²⁺] is too high, undesired PCR products may be seen. In some embodiments the magnesium concentration may be optimized by supplementing magnesium concentration in 0.1mM or 0.5mM increments up to about 5 mM.

Buffers used in accordance with the disclosure may contain additives such as surfactants, dimethyl sulfoxide (DMSO), glycerol, bovine serum albumin (BSA) and polyethylene glycol (PEG), as well as others familiar to those skilled in the art. Nucleotides are generally deoxyribonucleoside triphosphates, such as deoxyadenosine triphosphate (dATP), deoxycytidine triphosphate (dCTP), deoxyguanosine triphosphate (dGTP), and deoxythymidine triphosphate (dTTP), which are also added to a reaction adequate amount for amplification of the target nucleic acid. In some embodiments, the concentration of one or more dNTPs (e.g., dATP, dCTP, dGTP, dTTP) is from about 10 µM to about 500µM which may depend on the length and number of PCR products produced in a PCR reaction.

In some embodiments, the primers used in accordance with the disclosure are modified. The primers may be designed to bind with high specificity to only their intended target (e.g., a particular SNV) and demonstrate high discrimination against further nucleotide sequence differences. The primers may be modified to have a particular calculated melting temperature (Tm), for example a melting temperature ranging from 46 °C to 64 °C. To design primers with desired melting temperatures, the length of the primer may be varied and/or the GC content of the primer may be varied. Typically, increasing the GC content and/or the length of the primer will increase the Tm of the primer. Conversely, decreasing the GC content and/or the length of the primer will typically decrease the Tm of the primer. It should be appreciated that the primers may be modified by intentionally incorporating mismatch(es) with respect to the target in order to detect a particular SNV (or other form of sequence non-identity) over another with high sensitivity. Accordingly, the primers may be modified by incorporating one or more mismatches with respect to the specific sequence (e.g., a specific SNV) that they are designed to bind.

In some embodiments, the concentration of primers used in the PCR reaction may be modified or optimized. In some embodiments, the concentration of a primer (e.g., a forward or reverse primer) in a PCR reaction may be, for example, about 0.05 µM to about 1 µM. In particular embodiments, the concentration of each primer is about 1 nM to about 1 µM. It should be appreciated that the primers in accordance with the disclosure may be used at the same or different concentrations in a PCR reaction. For example, the forward primer of a primer pair may be used at a concentration of 0.5 µM and the reverse primer of the primer pair may be used at 0.1 µM. The concentration of the primer may be based on factors including, but not limited to, primer length, GC content, purity, mismatches with the target DNA or likelihood of forming primer dimers.

In some embodiments, the thermal profile of the PCR reaction is modified or optimized. Non-limiting examples of PCR thermal profile modifications include denaturation temperature and duration, annealing temperature and duration and extension time.

The temperature of the PCR reaction solutions may be sequentially cycled between a denaturing state, an annealing state, and an extension state for a predetermined number of cycles. The actual times and temperatures can be enzyme, primer, and target dependent. For any given reaction, denaturing states can range in certain embodiments from about 70 °C to about 100 °C. In addition, the annealing temperature and time can influence the specificity and efficiency of primer binding to a particular locus within a target nucleic acid and may be important for particular PCR reactions. For any given reaction, annealing states can range in certain embodiments from about 20 °C to about 75 °C. In some embodiments, the annealing state can be from about 46 °C to 64°C. In certain embodiments, the annealing state can be performed at room temperature (e.g., from about 20 °C to about 25 °C).

Extension temperature and time may also impact the allele product yield. For a given enzyme, extension states can range in certain embodiments from about 60 °C to about 75 °C.

Quantification of the amounts of the alleles from a quantification assay as provided herein can be performed as provided herein or as otherwise would be apparent to one of ordinary skill in the art. As an example, amplification traces are analyzed for consistency and robust quantification. Internal standards may be used to translate the cycle threshold to amount of input nucleic acids (e.g., DNA). The amounts of alleles can be computed as the mean of performant assays and can be adjusted for genotype. The wide range of efficient amplifications shows successful detection of low concentration nucleic acids.

It has been found that the methods provided herein can be used to detect low-level nucleic acids, such as non-native nucleic acids, in a sample. Accordingly, the methods provided herein can be used on samples where detection of relatively rare nucleic acids is needed. In some embodiments, any one of the methods provided herein can be used on a sample to detect non-native nucleic acids that are at least about 0.5% in the sample relative to total nucleic acids, such as total cf-DNA. In some embodiments, any one of the methods provided herein can be used on a sample to detect non-native nucleic acids that are at least 1% in the sample. In some embodiments, any one of the methods provided herein can be used on a sample to detect non-native nucleic acids that are at least about 1.3% in the sample. In some embodiments, any one of the methods provided herein can be used on a sample to detect non-native nucleic acids that are at least about 1.5% in the sample. In some embodiments, any one of the methods provided herein can be used on a sample to detect non-native nucleic acids that are at least about 2%, 3%, 4%, 5% or 10% in the sample.

Because of the ability to determine amounts of non-native nucleic acids, even at low levels, the methods and compositions provided herein can be used to assess a risk in a subject, such as a transplant recipient. A "risk" as provided herein, refers to the presence or absence or progression of any undesirable condition in a subject (such as a transplant recipient), or an increased likelihood of the presence or absence or progression of such a condition, e.g., transplant rejection. The condition can be any one of the conditions provided herein. As provided herein "increased risk" refers to the presence or progression of any undesirable condition in a subject or an increased likelihood of the presence or progression of such a condition. As provided herein, "decreased risk" refers to the absence of any undesirable condition or progression in a subject or a decreased likelihood of the presence or progression (or increased likelihood of the absence or nonprogression) of such a condition.

As an example, early detection of rejection following implantation of a transplant (e.g., a heart transplant) can facilitate treatment and improve clinical outcomes. Transplant rejection remains a major cause of graft failure and late mortality and generally requires lifelong surveillance monitoring. Treatment of transplant rejections with immunosuppressive therapy has been shown to improve treatment outcomes, particularly if rejection is detected early. Transplant rejection is typically monitored using a catheter-based endomyocardial biopsy (EMB). This invasive procedure, however, is associated with risks and discomfort for a patient, and may be particularly disadvantageous for pediatric patients. Accordingly, provided herein are sensitive, specific, cost effective, and non-invasive techniques for the surveillance of subjects, such as transplant recipients. Such techniques have been found to allow for the detection of transplant rejection at an early stage. Such techniques can also be used to monitor organ recovery and in the selection and monitoring of a treatment or therapy, such as an anti-rejection treatment or anti-infection treatment, thus improving a patient's recovery and increasing survival rates.

Accordingly, in the methods provided, the subject is a recipient of a transplant, and the risk is a risk associated with the transplant. In some embodiments of any one of the methods provided, the risk associated with the transplant is risk of transplant rejection, an anatomical problem with the transplant, or injury to the transplant. In some embodiments of any one of the methods provided, the injury to the transplant is initial or ongoing injury. In some embodiments of any one of the methods provided, the risk associated with the transplant is an acute condition or a chronic condition. In some embodiments of any one of the methods provided, the acute condition is transplant rejection including cellular rejection or antibody-mediated rejection. In some embodiments of any one of the methods provided, the chronic condition is graft vasculopathy. In some embodiments of any one of the methods provided, the risk associated with the transplant is indicative of the severity of the injury. In some embodiments of any one of the methods provided, the risk associated with the transplant is risk or status of an infection.

As used herein, "transplant" refers to the moving of an organ or tissue from a donor to a recipient for the purpose of replacing the recipient's damaged or absent organ or tissue. The transplant may be of one organ or more than one organ. In some embodiments, the term "transplant" refers to a transplanted organ or organs, and such meaning will be clear from the context the term is used. Examples of organs that can be transplanted include, but are not limited to, the heart, kidney(s), kidney, liver, lung(s), pancreas, intestine etc. Any one of the methods provided herein may be used on a sample from a subject that has undergone a transplant of any one or more of the organs provided herein. In some embodiments, the transplant is a heart transplant.

The risk in a recipient of a transplant can be determined, for example, by assessing the amount of non-native cf-DNA, such as donor-specific cell-free-DNA (DS cf-DNA), a biomarker for cellular injury related to transplant rejection. DS cf-DNA refers to DNA that presumably is shed from the transplanted organ, the sequence of which matches (in whole or in part) the genotype of the donor who donated the transplanted organ. As used herein, DS cf-DNA may refer to certain sequence(s) in the DS cf-DNA population, where the sequence is distinguishable from the recipient cf-DNA (e.g., having a different sequence at a particular nucleotide location(s)), or it may refer to the entire DS cf-DNA population.

The risk in a recipient of a transplant can be determined, for example, by assessing the amount of non-native cf-DNA, such as donor-specific cell-free DNA, as described herein using any one of the methods provided in combination with an assessment of the amount of total cell-free DNA, such as in ng/ml plasma. Thus, any one of the methods provided herein can include a step of obtaining the level of total cell-free DNA, such as in ng/ml in the subject. Such methods, in some embodiments, further includes assessing a risk associated with the transplant in the subject based on the combination of the amount of donor-specific cell-free DNA and total cell-free DNA in the subject. Methods for determining total cell-free DNA in the subject are known in the art. In some embodiments of any one of the methods provided herein, the total cell-free DNA is determined with TAQMAN^{™} Real-time PCR using RNase P as a target.

In some embodiments, any one of the methods provided herein can comprise correlating an increase in non-native nucleic acids and/or an increase in the ratio, or percentage, of non-native nucleic acids relative to native nucleic acids, with an increased risk of a condition, such as transplant rejection. In some embodiments of any one of the methods provided herein, correlating comprises comparing a level (e.g., concentration, ratio or percentage) of non-native nucleic acids to a threshold value to identify a subject at increased or decreased risk of a condition. In some embodiments of any one of the methods provided herein, a subject having an increased amount of non-native nucleic acids compared to a threshold value is identified as being at increased risk of a condition. In some embodiments of any one of the methods provided herein, a subject having a decreased or similar amount of non-native nucleic acids compared to a threshold value is identified as being at decreased risk of a condition.

As used herein, "amount" refers to any quantitative value for the measurement of nucleic acids and can be given in an absolute or relative amount. Further, the amount can be a total amount, frequency, ratio, percentage, etc. As used herein, the term "level" can be used instead of "amount" but is intended to refer to the same types of values.

"Threshold" or "threshold value", as used herein, refers to any predetermined level or range of levels that is indicative of the presence or absence of a condition or the presence or absence of a risk. The threshold value can take a variety of forms. It can be single cut-off value, such as a median or mean. It can be established based upon comparative groups, such as where the risk in one defined group is double the risk in another defined group. It can be a range, for example, where the tested population is divided equally (or unequally) into groups, such as a low-risk group, a medium-risk group and a high-risk group, or into quadrants, the lowest quadrant being subjects with the lowest risk and the highest quadrant being subjects with the highest risk. The threshold value can depend upon the particular population selected. For example, an apparently healthy population will have a different 'normal' range. As another example, a threshold value can be determined from baseline values before the presence of a condition or risk or after a course of treatment. Such a baseline can be indicative of a normal or other state in the subject not correlated with the risk or condition that is being tested for. In some embodiments, the threshold value can be a baseline value of the subject being tested. Accordingly, the predetermined values selected may take into account the category in which the subject falls. Appropriate ranges and categories can be selected with no more than routine experimentation by those of ordinary skill in the art.

Changes in the levels of non-native nucleic acids can also be monitored over time. For example, a change from a threshold value (such as a baseline) in the amount, such as ratio or percentage, of non-native nucleic acids can be used as a non-invasive clinical indicator of risk, e.g., risk associated with transplant. This can allow for the measurement of variations in a clinical state and/or permit calculation of normal values or baseline levels. In organ transplantation, this can form the basis of an individualized non-invasive screening test for rejection or a risk of a condition associated thereto. Generally, as provided herein, the amount, such as the ratio or percent, of non-native nucleic acids can be indicative of the presence or absence of a risk associated with a condition, such as risk associated with a transplant, such as rejection, in the recipient, or can be indicative of the need for further testing or surveillance. In some embodiments, for transplant recipients, this amount in combination with the total amount of cell-free DNA is indicative of the risk. In one embodiment of any one of the methods provided herein, the method may further include an additional test(s) for assessing a condition, such as transplant rejection, transplant injury, etc. The additional test(s) may be any one of the methods provided herein. In some embodiments, for transplant recipients, the additional test is a determination of the amount of total cell-free DNA in a sample from the subject.

In some embodiments of any one of the methods provided herein in regard to a heart transplant recipient, such threshold is 1%, wherein a level above 1% is indicative of an increased risk and wherein a level at or below 1% is indicative of a decreased risk. In some embodiments of any one of the methods provided herein in regard to a heart transplant recipient, such threshold is 1.3%, wherein a level above 1.3% is indicative of an increased risk and wherein a level at or below 1.3% is indicative of a decreased risk. In some embodiments of any one of the methods provided herein in regard to a heart transplant recipient, such threshold is 1.5%, wherein a level above 1.5% is indicative of an increased risk and wherein a level at or below 1.5% is indicative of a decreased risk. In some embodiments of any one of the methods provided herein in regard to a heart transplant recipient, such threshold is 2%, wherein a level above 2% is indicative of an increased risk and wherein a level at or below 2% is indicative of a decreased risk.

In some embodiments of any one of the methods provided herein, where a non-native nucleic acid amount, such as ratio or percentage, is determined to be above a threshold value, any one of the methods provided herein can further comprise performing another test on the subject or sample therefrom. Such other tests can be any other test known by one of ordinary skill in the art to be useful in determining the presence or absence of a risk, e.g., in a transplant recipient. In some embodiments, the other test is any one of the methods provided herein. In some embodiments of any one of the methods provided herein, the subject is a transplant recipient and the other test is a determination of the level of BNP and/or troponin in the transplant recipient. In other embodiments of any one of the methods provided herein, the other test in addition to the level of BNP and/or troponin or in place thereof is an echocardiogram.

In some embodiments of any one of the methods disclosed herein, the subject is a subject for fetal testing (not part of the presently claimed invention). Other tests include those that determine other clinical measures of fetal well-being, including ultrasound biometrical parameters, fetal heart rate, arterial pressure, amniotic fluid levels, bowel dilation, development of hydrops, resolution of hydrops after intervention and fetal loss. In some embodiments, the pH, PaO₂, PaCO₂, and/or blood lactate levels are assessed in the fetus and/or the pregnant subject. The methods provided and/or the additional test(s) can be performed at any of a number of time points during pregnancy, for example, time points can include during gestation, during progression of a condition, such as a disease, at fetal intervention, and upon resolution of hydrops after intervention. The determination may be performed instead of or in addition to other tests currently used to assess the condition of a fetus. The ability to detect early risk to a fetus, such as with a non-invasive method, can offer early intervention and better patient outcomes.

In some embodiments of any one of the methods provided herein, where the non-native nucleic acid amount, such as the ratio or percentage, is determined to be less than a threshold value such as 1% or 1.3% or 1.5% or 2%, such as for a transplant subject, no further testing is needed or recommended to the subject and/or no treatment is needed or suggested to the subject. While in some embodiments of any one of the methods provided herein, it may be determined that there is an increased risk in the transplant recipient when the amount of the non-native nucleic acid (e.g., ratio or percentage) in a sample obtained from the recipient is greater than 1% or 1.3% or 1.5% or 2%, although it should be appreciated that other thresholds may be utilized as embodiments of the invention are not limited in this respect. In some embodiments, the method may further comprise further testing or recommending further testing to the subject and/or treating or suggesting treatment to the subject. In some of these embodiments, the further testing is any one of the methods provided herein.

In some of these embodiments, the treating is an anti-rejection treatment or anti-infection treatment. In some embodiments, the information is provided in written form or electronic form. In some embodiments, the information may be provided as computer-readable instructions. In some embodiments, the information is provided orally.

Anti-rejection therapies include, for example, the administration of an immunosuppressive to a transplant recipient. Immunosuppressives include, but are not limited to, corticosteroids (e.g., prednisolone or hydrocortisone), glucocorticoids, cytostatics, alkylating agents (e.g., nitrogen mustards (cyclophosphamide), nitrosoureas, platinum compounds, cyclophosphamide (Cytoxan)), antimetabolites (e.g., folic acid analogues, such as methotrexate, purine analogues, such as azathioprine and mercaptopurine, pyrimidine analogues, and protein synthesis inhibitors), cytotoxic antibiotics (e.g., dactinomycin, anthracyclines, mitomycin C, bleomycin, mithramycin), antibodies (e.g., anti-CD20, anti-IL-1, anti-IL-2Ralpha, anti-T-cell or anti-CD-3 monoclonals and polyclonals, such as Atgam, and Thymoglobuline), drugs acting on immunophilins, ciclosporin, tacrolimus, sirolimus, interferons, opiods, TNF-binding proteins, mycophenolate, fingolimod and myriocin. In some embodiments, anti-rejection therapy comprises blood transfer or marrow transplant. Therapies can also include therapies for treating systemic conditions, such as sepsis. The therapy for sepsis can include intravenous fluids, antibiotics, surgical drainage, early goal directed therapy (EGDT), vasopressors, steroids, activated protein C, drotrecogin alfa (activated), oxygen and appropriate support for organ dysfunction. This may include hemodialysis in kidney failure, mechanical ventilation in pulmonary dysfunction, transfusion of blood products, and drug and fluid therapy for circulatory failure. Ensuring adequate nutrition-preferably by enteral feeding, but if necessary, by parenteral nutrition-can also be included particularly during prolonged illness. Other associated therapies can include insulin and medication to prevent deep vein thrombosis and gastric ulcers.

In some embodiments, wherein infection is indicated, therapies for treating a recipient of a transplant can also include therapies for treating a bacterial, fungal and/or viral infection. Such therapies include antibiotics. Other examples include, but are not limited to, amebicides, aminoglycosides, anthelmintics, antifungals, azole antifungals, echinocandins, polyenes, diarylquinolines, hydrazide derivatives, nicotinic acid derivatives, rifamycin derivatives, streptomyces derivatives, antiviral agents, chemokine receptor antagonist, integrase strand transfer inhibitor, neuraminidase inhibitors, NNRTIs, NS5A inhibitors, nucleoside reverse transcriptase inhibitors (NRTIs), protease inhibitors, purine nucleosides, carbapenems, cephalosporins, glycylcyclines, leprostatics, lincomycin derivatives, macrolide derivatives, ketolides, macrolides, oxazolidinone antibiotics, penicillins, beta-lactamase inhibitors, quinolones, sulfonamides, and tetracyclines. Other such therapies are known to those of ordinary skill in the art. Any one of the methods provided herein can include administering or suggesting an anti-infection treatment to the subject (including providing information about the treatment to the subject, in some embodiments). In some embodiments, an anti-infection treatment may be a reduction in the amount or frequency in an immunosuppressive therapy or a change in the immunosuppressive therapy that is administered to the subject. Other therapies are known to those of ordinary skill in the art.

The methods disclosed herein can also be used to identify and/or monitor a variety of fetal conditions (not part of the presently claimed invention). As used herein, a "fetal condition" or "condition of a fetus" refers to any health condition or assessment of well-being that may change over time. In some embodiments of any one of the methods disclosed herein, the method is not for determining the presence or absence of fetal aneuploidy, the gender of the fetus or the Rh blood type of the fetus. In some embodiments of any one of the methods disclosed herein, the condition of the fetus is the presence or absence or level of fetal distress (or fetal compromise).

The approach disclosed herein includes the detection and quantification of cell free DNA of fetal origin in a maternal sample. It has been found that the fraction (or percent or ratio) of cf-DNA in maternal blood varies as a function of fetal health and can be compared to threshold such as baseline values to assess fetal health. Fetuses can undergo cellular injury during distress from, for example, anatomic, metabolic, or issues of maternal environment that can result in fetal hydrops, a compromised newborn, prematurity or spontaneously terminated pregnancy. Fetal conditions include gastroschisis, fetal cardiac arrhythmias, congenital pulmonary adenomatoid malformations, Semilobar holoprosencephaly, fetal left heart syndrome, congenital defects (neuro, heart and others), low metabolic rate, fetal demise and twin-twin transfusion syndrome. The techniques provided can have broader applicability to detect fetal compromise in still other conditions, such as chorioamnionitis, preterm labor, fetal cardiac arrhythmias (also associated with an increased risk of fetal hydrops and in utero demise), fetal bradycardia (which can occur from a variety of causes, most commonly from maternal collagen vascular diseases, such as Systemic Lupus (SLE)), large congenital pulmonary adenomatoid malformations (CPAMs), etc.

The methods disclosed herein, accordingly, can be used in a variety of situations to assess the condition of the fetus (not part of the presently claimed invention). For example, fetal well-being can be assessed in a fetus that has been diagnosed with a congenital anomaly, such as congenital heart disease. As another example, fetal well-being can be assessed in pregnant women with fetal gastroschisis and fetal bradycardia syndrome. In high or low risk pregnancies, the methods can allow for the recognition of fetal compromise for early intervention or delivery to prevent fetal loss. The methods, therefore, can assist in planning a woman's pregnancy and allow for medical intervention, appropriate timing of delivery, etc. Such methods can allow for the further understanding of pre-term labor and fetal demise and decrease the rate of prematurity and neonatal mortality, which unfortunately, remain unacceptably high. Accordingly, any one of the methods disclosed can be for use in any one of the conditions provided herein, such as the foregoing situations or for the foregoing subjects.

The methods disclosed can be used to monitor any pregnancy, where a fetus has or is suspected of having any one of the conditions provided herein. For example, levels or amounts, such as percentages, fractions or ratios, of fetal specific nucleic acids, such as cf-DNA, such as relative to total cf-DNA or non-fetal specific cf-DNA, can be determined using any one of the methods disclosed herein. It should also be appreciated that any one of the methods provided can include a step of correcting the results based on maternal weight and/or gestational age. The methods disclosed herein, therefore, can be used to identify and/or monitor fetal well-being over several time points during gestation and/or progression of disease. Such monitoring can also occur after fetal intervention.

Accordingly, the methods disclosed can be used to monitor low- as well as high-risk pregnancies, including intra-uterine growth restriction and maternal vascular diseases (not part of the presently claimed invention). "Low-risk pregnancy" refers to any pregnancy a clinician would deem to be at low risk for one or more complications or conditions associated with such complication(s). "High-risk pregnancy" is meant to refer to any pregnancy a clinician would deem at risk for one or more complications or conditions associated with such complication(s). For example, a pregnancy can be considered high-risk when there are potential complications that could affect the mother, the baby, or both. Further examples where a pregnancy may be indicated as high-risk include pregnancies where health problems exist (e.g., diabetes, cancer, high blood pressure, kidney disease (e.g., chronic pyelonephritis, chronic pyelonephritis and renal insufficiency), epilepsy). A pregnancy may also be deemed high risk if the mother uses alcohol or illegal drugs, or smokes; is younger than 17 or older than 35; has a history of multiple pregnancies, has a history of prior miscarriages; or where the fetus is found to have genetic conditions such as Down syndrome, or a heart, lung, or kidney problem. High risk pregnancies also include those who had prior or have current problems in pregnancy (e.g., preterm labor, preeclampsia or seizures (eclampsia)); those who have an infection (e.g., HIV, hepatitis C, cytomegalovirus (CMV), chickenpox, rubella, toxoplasmosis, or syphilis); or those taking certain medications (e.g., lithium, phenytoin (such as Dilantin), valproic acid (Depakene), or carbamazepine (such as Tegretol)). Pregnancies where the pregnant woman has certain health problems (e.g., heart valve problems, hypertension, sickle cell disease, asthma, lupus, or rheumatoid arthritis) can also be considered high risk. Other clinical indicators of high-risk pregnancies are known to those of ordinary skill in the art.

As used herein, a "baseline level" for such subjects can include an amount of fetal specific nucleic acids, such as fetal specific cf-DNA (such as the percent or ratio of fetal specific cf-DNA relative to the total cf-DNA or non-specific cf-DNA), from a sample from the subject taken at a time prior to a subsequent sample or at a time where the subject (e.g., the pregnant woman or fetus) was or was believed to be in good health, did not have or was not believed to have a condition provided herein, or did have or was believed to have a condition provided herein but the condition was or was believed to be at a stage that did not require treatment or intervention. In any one of the methods provided herein, decrease change relative to the baseline can be an indicator of fetal well-being. In any one of the methods provided herein changes in the difference relative to the baseline at two or more time points can be an indicator of fetal well-being. Accordingly, in any one of the methods disclosed herein the amount of fetal specific cf-DNA is determined at one or more time points, and the increase or decrease relative to one or more baselines values is determined. In any one of the methods disclosed herein the amount of fetal specific nucleic acids is determined at two or more time points, and the changes in the difference relative to one or more baseline values is determined. In any of the methods disclosed herein, the methods can further comprise a step of spiking in an internal standard at known quantities to aid in the quantitation of the specific nucleic acids. In any one of the methods disclosed herein for assessing fetal well being, an amount, such as the fraction of fetal specific nucleic acids, such as cf-DNA, that is less than 10% at at least 10 weeks gestation is indicative of increased risk.

Any one of the methods provided can include the step of providing information regarding a therapy or treatment to the subject based on the determined amount, such as the determination of an amount relative to a threshold, such as a baseline, or change in the amount relative to a threshold, such as a baseline. In some embodiments, the information includes written materials containing the information. Written materials can include the written information in electronic form. In any one of the methods provided herein, the method can further comprise recording the administration of a therapy, the providing of information for a therapy or the suggesting of a therapy to the subject. The therapy may be any one of the therapies or treatments provided herein or otherwise known to those of ordinary skill in the art. Alternatively, no change or no therapy or treatment may be determined and suggested based on the amount, and any one of the methods provided herein can include a step of providing such information to the subject.

For a pregnant subject (not part of the presently claimed invention), any one of the methods disclosed herein, therefore, can further include a step of performing or recommending fetal intervention (e.g. surgical procedure, administration of a drug) or delivery of the baby before an intrauterine death occurs for the pregnant woman. In some embodiments, the recommending comprises providing information regarding a suggested treatment, such as fetal intervention and/or delivery options to the pregnant woman. The information can be provided orally.

In still other embodiments, any one of the methods can be used to assess the efficacy of a therapy (or treatment) where improved values can indicate less of a need for the therapy, while worsening values can indicate the need for a therapy, a different therapy, or an increased amount of a therapy. Any one of the methods provided herein can include the step of evaluating the need or dose of a therapy based on the result of one or more comparisons at one or more time points.

It may be particularly useful to a clinician to have a report that contains the value(s) provided herein. In one aspect, therefore such reports are provided. Reports may be in oral, written (or hard copy) or electronic form, such as in a form that can be visualized or displayed. In some embodiments, the "raw" results for each assay as provided herein are provided in a report, and from this report, further steps can be taken to analyze the amount(s) of total nucleic acids (such as total cell-free DNA) and non-native nucleic acids (such as donor-specific cell-free DNA). In other embodiments, the report provides multiple values for the amounts of total nucleic acids (such as total cell-free DNA) and non-native nucleic acids (such as donor-specific cell-free DNA). From the amounts, in some embodiments, a clinician may assess the need for a treatment for the subject or the need to monitor the subject over time.

Accordingly, in any one of the methods provided herein, the method can include assessing the amount of total nucleic acids (such as total cell-free DNA) and non-native nucleic acids (such as donor-specific cell-free DNA) in the subject at another point in time or times. Such assessing can be performed with any one of the methods provided herein.

Methods for determining cell-free nucleic acids are provided herein or are otherwise known in the art. As a further example, real-time PCR may be used to determine an amount of total nucleic acids (such as total cell-free DNA). For example, in some embodiments of any one of the methods provided herein, the total nucleic acids (such as total cell-free DNA) is determined with TAQMAN^{™} Real-time PCR using RNase P as a target. Other methods would be apparent to those of ordinary skill in the art.

Any one of the methods provided herein can comprise extracting nucleic acids, such as cell-free DNA, from a sample obtained from a subject, such as a recipient of a transplant. Such extraction can be done using any method known in the art or as otherwise provided herein (see, e.g., Current Protocols in Molecular Biology, latest edition, or the QIAamp circulating nucleic acid kit or other appropriate commercially available kits). An exemplary method for isolating cell-free DNA from blood is described. Blood containing an anticoagulant such as EDTA or DTA is collected from a subject. The plasma, which contains cf-DNA, is separated from cells present in the blood (e.g., by centrifugation or filtering). An optional secondary separation may be performed to remove any remaining cells from the plasma (e.g., a second centrifugation or filtering step). The cf-DNA can then be extracted using any method known in the art, e.g., using a commercial kit such as those produced by Qiagen. Other exemplary methods for extracting cf-DNA are also known in the art (see, e.g., Cell-Free Plasma DNA as a Predictor of Outcome in Severe Sepsis and Septic Shock. Clin. Chem. 2008, v. 54, p. 1000-1007; Prediction of MYCN Amplification in Neuroblastoma Using Serum DNA and Real-Time Quantitative Polymerase Chain Reaction. JCO 2005, v. 23, p.5205-5210; Circulating Nucleic Acids in Blood of Healthy Male and Female Donors. Clin. Chem. 2005, v. 51, p.1317-1319; Use of Magnetic Beads for Plasma Cell-free DNA Extraction: Toward Automation of Plasma DNA Analysis for Molecular Diagnostics. Clin. Chem. 2003, v. 49, p. 1953-1955; Chiu RWK, Poon LLM, Lau TK, Leung TN, Wong EMC, Lo YMD. Effects of blood-processing protocols on fetal and total DNA quantification in maternal plasma. Clin Chem 2001;47:1607-1613; and Swinkels et al. Effects of Blood-Processing Protocols on Cell-free DNA Quantification in Plasma. Clinical Chemistry, 2003, vol. 49, no. 3, 525-526).

As used herein, the sample from a subject can be a biological sample. Examples of such biological samples include whole blood, plasma, serum, urine, etc. In some embodiments of any one of the methods provided herein, addition of further nucleic acids, e.g., a standard, to the sample can be performed.

In some embodiments of any one of the methods provided herein, a pre-amplification step is performed. An exemplary method of such an amplification is as follows, and such a method can be included in any one of the methods provided herein. Approximately 15 ng of cell free plasma DNA is amplified in a PCR using Q5 DNA polymerase with approximately 100 targets where pooled primers were at 6uM total. Samples undergo approximately 35 cycles. Reactions are in 25 ul total. After amplification, samples can be cleaned up using several approaches including AMPURE bead cleanup, bead purification, or simply ExoSAP-IT^{™}, or Zymo.

The present disclosure also provides methods for determining a plurality of SNV targets for use in any one of the methods provided herein or from which any one of the compositions of primers can be derived. A method of determining a plurality of SNV targets, in some embodiments comprises a) identifying a plurality of highly heterozygous SNVs in a population of individuals, b) designing one or more primers spanning each SNV, c) selecting sufficiently specific primers, d) evaluating multiplexing capabilities of primers, such as at a common melting temperature and/or in a common solution, and e) identifying sequences that are evenly amplified with the primers or a subset thereof.

As used herein, "highly heterozygous SNVs" are those with a minor allele at a sufficiently high percentage in a population. In some embodiments, the minor allele is at least 25%, 26%, 27%, 28%, 29%, 30%, 31%, 32%, 33%, 34% or 35% or more in the population. In any one of these embodiments, the minor allele is less than 50%, 49%, 45%, 44%, 43%, 42%, 41%, or 40% in the population. Such SNVs increase the likelihood of providing a target that is different between the native and non-native nucleic acids.

Primers can be designed to generally span a 70bp window but some other window may also be selected, such as one between 65 bps and 75 bps, or between 60bps and 80bps. Also, generally, it can be desired for the SNV to fall about in the middle of this window. For example, for a 70bp window, the SNV can be between bases 10-60 or 20-50, such as between bases 30-40. The primers as provided herein can be designed to be adjacent to the SNV.

As used herein, "sufficiently specific primers", were those that demonstrated discrimination between amplification of the intended allele versus amplification of the unintended allele. Thus, with PCR a cycle gap can be desired between amplification of the two. In one embodiment, the cycle gap can be at least a 5, 6, 7 or 8 cycle gap.

Further, sequences can be selected based on melting temperatures, generally those with a melting temperature of between 45-55 degrees C were selected as "moderate range sequences". Other temperature ranges may be desired and can be determined by one of ordinary skill in the art. A "moderate range sequence" generally is one that can be amplified in a multiplex amplification format within the temperature. In some embodiments, the GC% content can be between 30-70% or between 35%-65%, such as between 33-66%.

In one embodiment of any one of the methods provided herein, the method can further comprise excluding sequences associated with difficult regions. "Difficult regions" are any regions with content or features that make it difficult to reliably make predictions about a target sequence or are thought to not be suitable for multiplex amplification. Such regions include syndromic regions, low complexity regions, regions with high GC content or that have sequential tandem repeats. Other such features can be determined or are otherwise known to those of ordinary skill in the art.

In some embodiments of the method, provided herein, the primer pairs are designed to be compatible for use in a quantitative assay as provided herein. For example, the primer pairs are designed to prevent primer dimers and/or limit the number of off-target binding sites. It should be appreciated that the plurality of primer pairs of the method provided may be optimized or designed in accordance with any one of the methods described herein.

In any one of the compositions or kits disclosed herein a buffer may be provided. In some embodiments, the buffers contain additives such as surfactants, dimethyl sulfoxide (DMSO), glycerol, bovine serum albumin (BSA) and polyethylene glycol (PEG) or other PCR reaction additive. In any one of the compositions or kits disclosed herein a polymerase may be provided for example, the composition or kit may comprise E. coli DNA polymerase, Klenow fragment of E. coli DNA polymerase I, T7 DNA polymerase, T4 DNA polymerase, T5 DNA polymerase, Klenow class polymerases, Taq polymerase, Pfu DNA polymerase, Vent polymerase, bacteriophage 29, REDTaq^{™} Genomic DNA polymerase, or sequenase. In any one of the compositions or kits disclosed herein one or more dNTPs (e.g., dATP, dCTP, dGTP, dTTP) may be provided. In some embodiments, any one of the compositions or kits disclosed herein further comprises a probe (e.g., a TAQMAN^{™} probe).

A "kit," as used herein, typically defines a package or an assembly including one or more of the compositions as previously described. Any one of the kits disclosed herein may further comprise at least one reaction tube, well, chamber, or the like. Any one of the primers, primer systems (such as a set of primers for a plurality of targets) or primer compositions described herein may be provided in the form of a kit or comprised within a kit.

Each of the compositions of the kit may be provided in liquid form (e.g., in solution), in solid form (e.g., a dried powder), etc. A kit may, in some cases, include instructions in any form that are provided in connection with the compositions of the invention in such a manner that one of ordinary skill in the art would recognize that the instructions are to be associated with the compositions of the invention. The instructions may include instructions for performing any one of the methods provided herein. The instructions may include instructions for the use, modification, mixing, diluting, preserving, administering, assembly, storage, packaging, and/or preparation of the compositions and/or other compositions associated with the kit. The instructions may be provided in any form recognizable by one of ordinary skill in the art as a suitable vehicle for containing such instructions, for example, written or published, verbal, audible (e.g., telephonic), digital, optical, visual (e.g., videotape, DVD, etc.) or electronic communications (including Internet or web-based communications), provided in any manner.

Various aspects of the present invention may be used alone, in combination, or in a variety of arrangements not specifically discussed in the embodiments described in the foregoing and are therefore not limited in their application to the details and arrangement of components set forth in the foregoing description or illustrated in the drawings. For example, aspects described in one embodiment may be combined in any manner with aspects described in other embodiments.

Also, embodiments of the invention may be implemented as one or more methods, of which an example has been provided. The acts performed as part of the method(s) may be ordered in any suitable way. Accordingly, embodiments may be constructed in which acts are performed in an order different from illustrated, which may include performing some acts simultaneously, even though shown as sequential acts in illustrative embodiments.

Use of ordinal terms such as "first," "second," "third," etc., in the claims to modify a claim element does not by itself connote any priority, precedence, or order of one claim element over another or the temporal order in which acts of a method are performed. Such terms are used merely as labels to distinguish one claim element having a certain name from another element having a same name (but for use of the ordinal term).

The phraseology and terminology used herein is for the purpose of description and should not be regarded as limiting. The use of "including," "comprising," "having," "containing", "involving", and variations thereof, is meant to encompass the items listed thereafter and additional items.

Having described several embodiments of the invention in detail, various modifications and improvements will readily occur to those skilled in the art. Accordingly, the foregoing description is by way of example only, and is not intended as limiting. The following description provides examples of the methods provided herein.

### EXAMPLES

### Example 1 - MOMA Assay With Recipient and Donor Genotype Information

### SNV Target Selection

Identification of targets for multiplexing in accordance with the disclosure may include one or more of the following steps, as presently described. First, highly heterozygous SNPs were screened on several ethnic control populations (Hardy-Weinberg p > 0.25), excluding known difficult regions. Difficult regions include syndromic regions likely to be abnormal in patients and regions of low complexity, including centromeres and telomeres of chromosomes. Target fragments of desired lengths were then designed *in silico.* Specifically, two 20-26 bp primers spanning each SNP's 70 bp window were designed. All candidate primers were then queried to GCRh37 using BLAST. Those primers that were found to be sufficiently specific were retained, and monitored for off-target hits, particularly at the 3' end of the fragment. The off-target candidate hits were analyzed for pairwise fragment generation that would survive size selection. Selected primers were then subjected to an *in silico* multiplexing evaluation. The primers' computed melting temperatures and guanine-cytosine percentages (GC%) were used to filter for moderate range sequences. An iterated genetic algorithm and simulated annealing were used to select candidate primers compatible for 400 targets, ultimately resulting in the selection of 800 primers. The 800 primers were generated and physically tested for multiplex capabilities at a common melting temperature in a common solution. Specifically, primers were filtered based on even amplification in the multiplex screen and moderate read depth window. Forty-eight assays were designed for MOMA using the top performing multiplexed SNPs. Each SNP had a probe designed in WT/MUT at four mismatch choices; there were eight probes per assay. The new nested primers were designed within the 70 bp enriched fragments. Finally, the primers were experimentally amplified with known heterozygous individuals to evaluate amplification efficiency (8 probes x 48 assays in triplicate, using TAQMAN^{™}).

### A priori Genotyping Informativeness of each Assay

Using the known recipient and donor genotypes at each assayed SNP, a subset of informative assays was selected. Note that recipient homozygous sites can be used where the donor is any other genotype. Additionally, if the donor genotype is not known, it can be inferred, such as by using plasma data discrepancies, provided that clear recipient genotypes are available before transplant. Genotypes may also be learned through sequencing, SNP microarray, or application of a MOMA assay on known 0% (clean recipient) samples.

### Post Processing Analysis of Multiplex Assay Performance

Patient-specific MOMA probe biases were estimated across the experimental cohort. Selection iteratively was refined to make the final donor percent call. Further, automatic outlier detection provided patient-specific anomalous genomic regions.

### Reconstruction Experiment

The sensitivity and precision of the assay were evaluated using reconstructed plasma samples with known mixing ratios. Specifically, the ratios of 1:10, 1:20, 1:100, 1:200, and 1:1000 were evaluated.

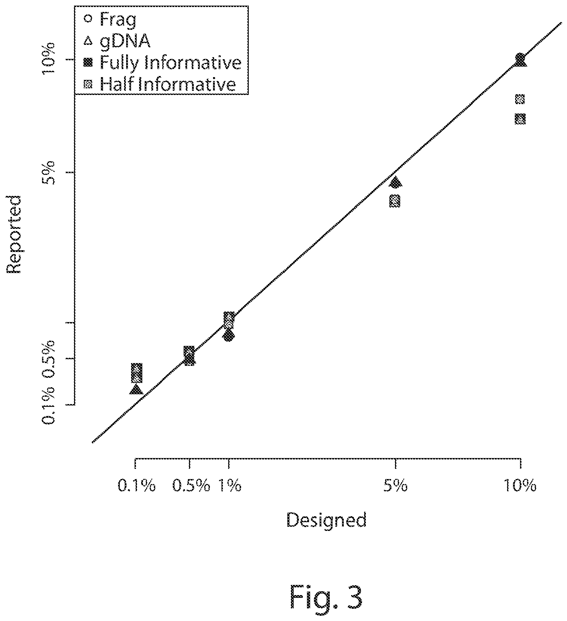

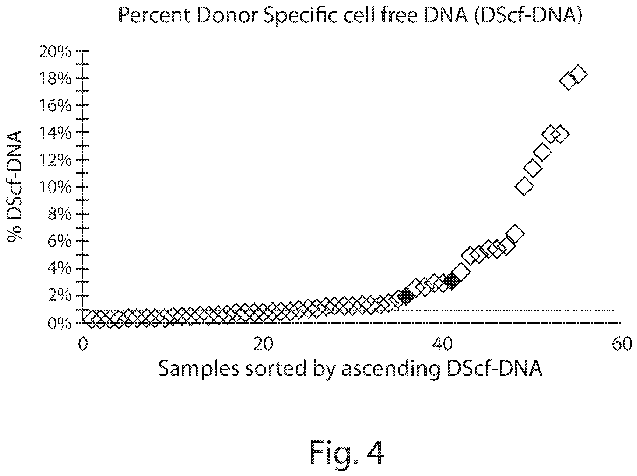

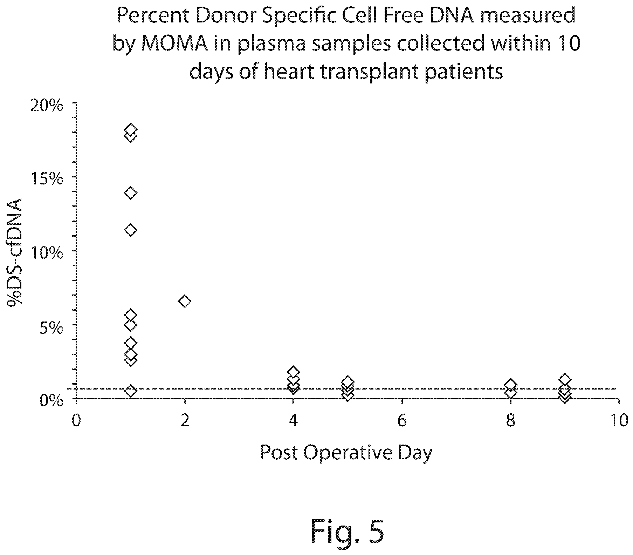

Results of the reconstruction experiment are shown in **Fig. 3****.** One target is fully informative where there is a homozygous donor against a homozygous recipient (shaded data points). The other target is half informative where there is a heterozygous donor against a homozygous recipient (open data points). In addition, plasma samples from transplant recipient patients were analyzed with a MOMA method (**Fig. 4**). All data comes from patients who have had biopsies. Dark points denote rejection. Further data shown in **Fig. 5** demonstrate that a MOMA method as provided herein worked with plasma samples. After transplant surgery, the donor percent levels dropped off. Generally, primers for 95 SNV targets as described herein were used for these experiments.

### Example 2 - MOMA Assay With Recipient but not Donor Genotype Information

To work without donor genotype information, the following procedure may be performed to infer informative assays and allow for quantification of donor-specific cell-free DNA in plasma samples. All assays were evaluated for performance in the full information scenario. This procedure thus assumed clean AA/AB/BB genotypes at each assay and unbiased behavior of each quantification. With recipient genotype, assays known to be homozygous in the recipient were selected. Any contamination was attributed to the donor nucleic acids, and the assay collection created a tri-modal distribution with three clusters of assays corresponding to the non-, half, and fully-informative assays. With sufficient numbers of recipient homozygous assays, the presence of donor fully informative assays can be assumed.

If the recipient genotype is homozygous and known, then if a measurement that is not the recipient genotype is observed, the probes which are truly donor-homozygous will have the highest cluster and equal the guess whereas those that are donor heterozygous will be at half the guess. A probability distribution can be plotted and an expectation maximization algorithm (EM) can be employed to infer donor genotype. Such can be used to infer the donor genotype frequency in any one of the methods provided herein. Accordingly, an EM algorithm was used to infer the most likely donor genotypes at all assayed SNV targets. With inferred donor genotypes, quantification may proceed as in the full-information scenario. EM can begin with the assumption that the minor allele ratio found at an assay follows a tri-modal distribution, one for each combination of recipient and donor, given all assays are "AA" in the recipient (or flipped from "BB" without loss of generality). With all donor genotypes unknown, it is possible to bootstrap from the knowledge that any assays exhibiting nearly zero minor allele are donor AA, and the highest is donor BB. Initial guesses for all donor genotypes were recorded, and the mean of each cluster calculated. Enforcing that the donor BB assays' mean is twice that of the donor AB restricts the search. The algorithm then reassigns guessed donor genotypes based on the clusters and built-in assumptions. The process was iterative until no more changes were made. The final result is a set of the most likely donor genotypes given their measured divergence from the background. Generally, every target falls into the model; a result may be tossed if between groups after maximization.

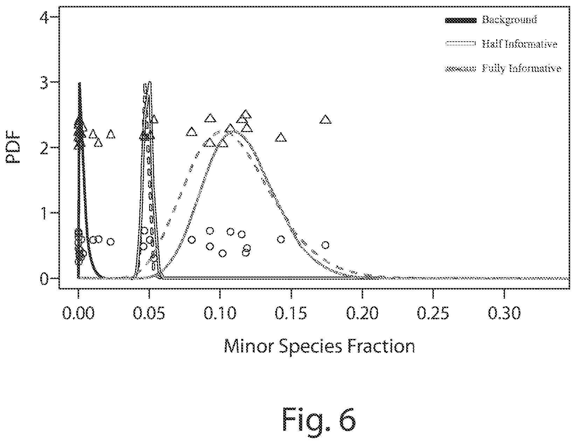

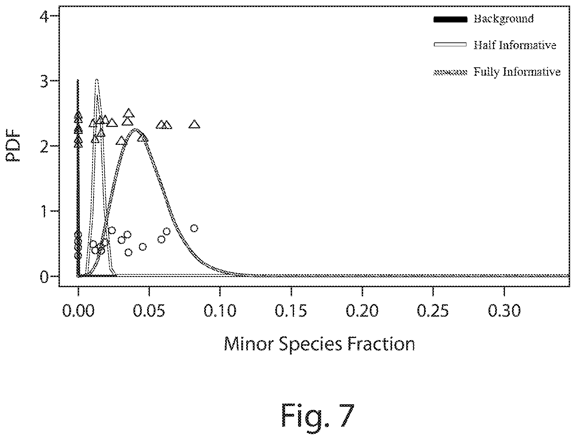

**Figs. 6** and **7** show exemplary results from plasma samples handled in this manner. The x-axis is the donor% for any assay found recipient homozygous. The rows of points represent individual PCR assay results. The bottom-most row of circles represents the initial guess of donor genotypes, some AA, some A/B and some BB. Then the solid curves were drawn representing beta distributions centered on the initial assays, dotted for homozygous (fully informative) and white for heterozygous (half informative) with black curves representing the distribution of non-informative assays or background noise. The assays were re-assigned updated guesses in the second row. The second row's curves use dashed lines. The top row is the final estimate because no change occurred. Double the peak of the white dashed curve corresponds to the maximum likelihood donor% call, at around 10%, or equal to the mean of the dotted curve.

A reconstruction experiment (Recon1) using DNA from two individuals was created at 10%, 5%, 1%, 0.5%, and 0.1%. All mixes were amplified with a multiplex library of targets, cleaned, then quantitatively genotyped using a MOMA method. The analysis was performed with genotyping each individual in order to know their true genotypes. Informative targets were determined using prior knowledge of the genotype of the major individual (looking for homozygous sites), and where the second individual was different, and used to calculate fractions (percentage) using informative targets. The fractions were then calculated (depicted in black to denote "With Genotype" information).

A second reconstruction experiment (Recon2), beginning with two individuals, major and minor, was also created at 10%, 5%, 1%, 0.5%, and 0.1%. All mixes were amplified with the multiplex library of targets, cleaned, and then quantitatively genotyped using a MOMA method. The analysis was performed by genotyping each individual in order to know their true genotypes. Informative targets were determined using prior knowledge of the genotype of the second individual as described above. The fractions were then calculated (depicted in black to denote "With Genotype" information).

These reconstructions were run again the next day (Recon3).

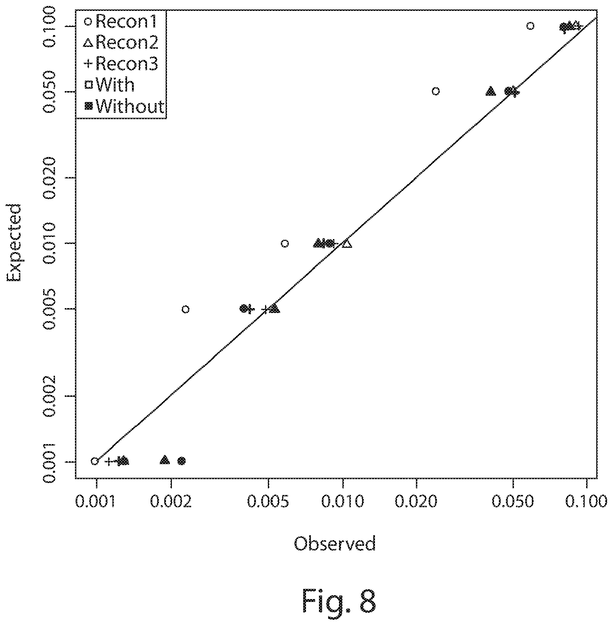

The same reconstruction samples (Recon 1,2,3) were then analyzed again only using the genotyping information available for the first individual (major DNA contributor). Genotyping information from the second individual (minor DNA contributor) was not used. Approximately 38-40 targets were used to calculate fractions without genotyping (simulating without donor); they are presented as shaded points (**Fig. 8**). It was found that each target that was recipient homozygous was generally useful. The circles show a first estimate, a thresholding; those on the right were thought to be fully informative and those on the left, not. The triangles along the top were the same targets, but for the final informativity decisions they were recolored.

### Example 3 - MOMA Assay With Native but without Non-Native Genotype Information (not in accordance with the presently claimed invention)

Targets generally are identified as either non-informative or informative. In fetal, all targets are half-informative when the baby is related to the mother. In a surrogate pregnancy, the targets are half and fully informative. Continuing in the context of a related pregnancy, all targets are either non-informative or half. The half-informative show a heterozygous behavior such that a target appearing 5% foreign represents a 10% overall proportion. A non-informative target shows nearly 0% at the background noise level. An expectation maximization (EM) algorithm can used to infer the most likely assignments for each target, based on the assumption that the informative targets share a common distribution. A distribution such as a "beta" distribution can be used to give a continuous probability distribution between 0 and 1. On the assumption that all targets are either a background-noise distribution or the fetal-specific proportion, an EM algorithm fills in the unknown target categorization.

For example, consider a set of ten targets, each with a called fetal-specific percentage. The first guess is that any targets calling less than the 25th percentile are background noise and the others are half-informative. With the first guess in place, the EM may begin. Next, the maximization step is performed. A distributional model, such as a beta distributional model, is fit over each point group, minimizing the sum of the log-likelihood function of the data points to a two-dimensional model. The beta distribution is defined by two shape parameters. In total, four shape parameters are collected (two per target informativity group). The maximum likelihood estimate of the non-native% is equal to twice the mean of the beta model of the half-informative group. In the subsequent expectation step, all of the targets are evaluated to find to which group each has a higher likelihood of belonging. Targets are reassigned to non- or half-informative groups based on which model has the higher likelihood. If any targets have changed groups, the maximization step is repeated, followed by the expectation step again. Upon convergence, the EM is complete and reports the maximum likelihood informativity categorization of all targets and the final model parameters.

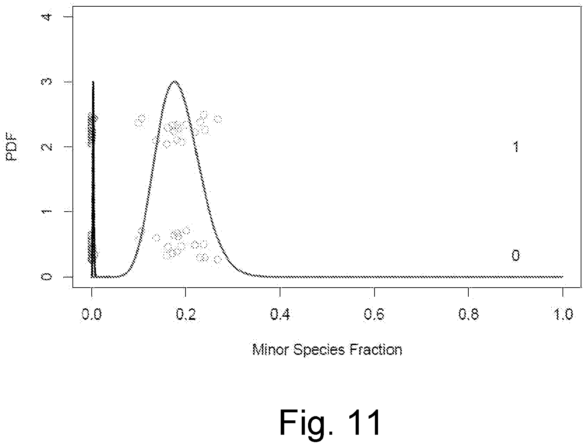

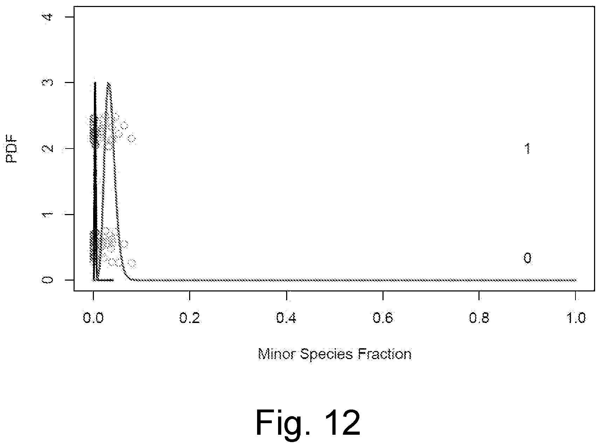

Examples of inferring a genotype are provided in **Figs. 11** and **12****,** where EM was used to determine fetal-specific calculations, where the father's genotype was not known. The method was able to distinguish non-informative from half-informative sites. These are then multiplied by 2 to determine the fetal fraction.

In addition, using a method provided herein, a low fetal fraction was found to correlate with a fetal demise subject. It was also found that a fetal fraction of 1-10% after fetal gestational age of 10 week indicated developmental pathology.

### Example 4 - MOMA cf-DNA Assay

### Principles and Procedures of a MOMA cf-DNA Assay

This exemplary assay is designed to determine the percentage of DS cf-DNA present in a transplant recipient's blood sample. In this embodiment, the recipient's blood sample is collected in an EDTA tube and centrifuged to separate the plasma and buffy coat. The plasma and buffy coat can be aliquoted into two separate 15 mL conical tubes and frozen. The plasma sample can be used for quantitative genotyping (qGT), while the buffy coat can be used for basic genotyping (bGT) of the recipient. In addition to the transplant recipient's blood sample, a small piece of discarded tissue or blood sample from the donor can be used for basic genotyping.

The first step in the process can be to extract cell free DNA from the plasma sample (used for qGT) and genomic DNA (gDNA) from the buffy coat, whole blood, or tissue sample (used for bGT). The total amount of cfDNA can be determined by qPCR and normalized to a target concentration. This process is known as a cfDNA Quantification. gDNA can be quantified using UV-spectrophotometry and normalized. Fifteen ng of DNA generally provides accurate and valid results.

The normalized patient DNA can be used as an input into a highly-multiplexed library PCR amplification reaction containing, for example, 96 primer pairs, each of which amplify a region including one of the MOMA target sites. The resulting library can be used as the input for either the bGT or qGT assay as it consists of PCR amplicons having the MOMA target primer and probe sites. This step can improve the sensitivity of the overall assay by increasing the copy number of each target prior to the highly-specific qPCR amplification. Controls and calibrators/standards can be amplified with the multiplex library alongside patient samples. Following the library amplification, an enzymatic cleanup can be performed to remove excess primers and unincorporated deoxynucleotide triphosphates (dNTPs) to prevent interference with the downstream amplification.

In a parallel workflow the master mixes can be prepared and transferred to a 384-well PCR plate. The amplified samples, controls, and calibrators/standards can then be diluted with the library dilution buffer to a predetermined volume and concentration. The diluted samples and controls can be aliquoted to a 6-well reservoir plate and transferred to the 384-well PCR plate using an acoustic liquid handler. The plate can then be sealed and moved to a real-time PCR amplification and detection system.

MOMA can perform both the basic and quantitative genotyping analyses by targeting biallelic SNPs that are likely to be distinct between a transplant donor and recipient making them highly informative. The basic genotyping analysis can label the recipient and donor with three possible genotypes at each target (e.g. homozygous REF, heterozygous REF and VAR, and homozygous VAR). This information can be used for the quantitative genotyping analysis, along with standard curves, to quantitate to the allele ratio for each target, known as a minor-species proportion. The median of all informative and quality-control passed allele ratios can be used to determine the % of DS cfDNA.

### Variability of a MOMA cf-DNA Assay

Following MOMA analyses run as described above, one control sample was processed repeatedly. This sample was a constructed mixture, approximately one percent donor DNA and 99% recipient DNA. This constitutes the 'positive control' (or PTC), and it was run repeatedly to ensure the overall system was stable. In this time period, several hundred samples were run. The PTC was re-run on 17 batches, presenting an opportunity to investigate the variability of the MOMA cf-DNA assay, and to illustrate how the variability can be influenced by the number of sub-assay (targets) in use.

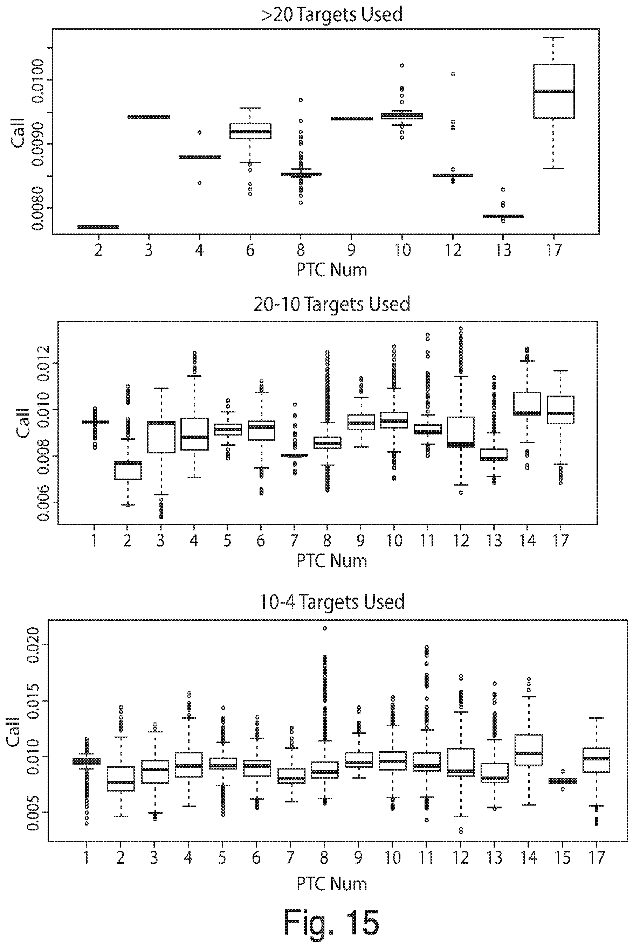

In the multi-assay platform, each 'target' independently evaluates the donor fraction. With knowledge of recipient and donor genotype (or inference thereof), it is possible to know which targets will be distinct between individuals and therefore informative to an overall average cf-DNA result. Each target has variability, and precision is gained by using a collection and reporting the median. Therefore, using more targets generally can allow for greater accuracy in the final result (see **Fig. 15**). The results provided below further illustrate the correlation between targets-in-use and the accuracy of the result.

The PTC sample was subdivided into single-use tubes and each processed within DNA-extraction batches, approximately one per 30 true study/patient samples. Therefore the PTC was re-processed independently about once or twice a day through the course of the study. Each has a slightly different resulting call, but all were around the intended 1%.

The per-target dataset was collected and down-sampled to empirically simulate the effect of having ever-fewer informative targets. The PTC had 36 targets potentially informative of the 96 candidate targets. In the context of an organ transplant recipient, the maximal set of informative targets for this example is the number of targets that are simultaneously not heterozygous in the recipient genome and distinct in the donor genome. In the context of a pregnancy the non-native cf-DNA is that of the fetus, sharing genotypes with its parents. In the case of a fetus that is the offspring of the mother, the maximal set of informative targets is the number of targets that are simultaneously not heterozygous in the mother's genome and distinct in the fetal genome.

The PTC had on average 21 targets both informative and robustly quantifiable on each run. Variability in the usable target number is derived from the other run parameters and calibration samples reducing the targets from the maximum. From a collection of 398 patient samples, the number of informative targets was 28 (23-31) (median, interquartile range), and the number of targets both informative and robustly quantifiable was 16 (13-20).

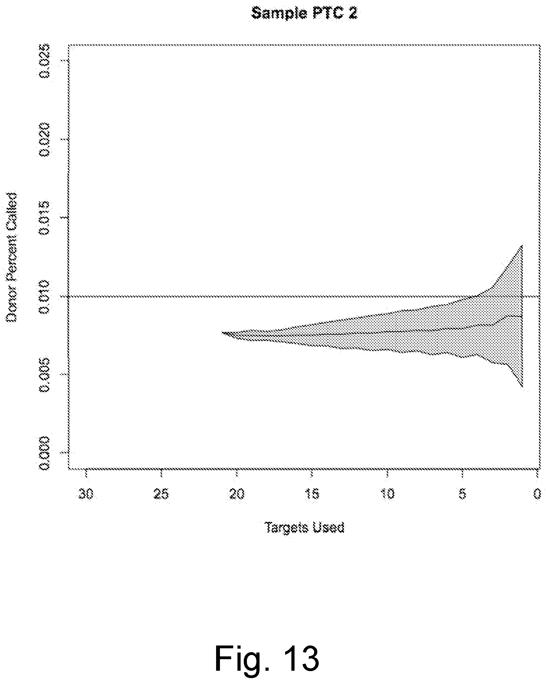

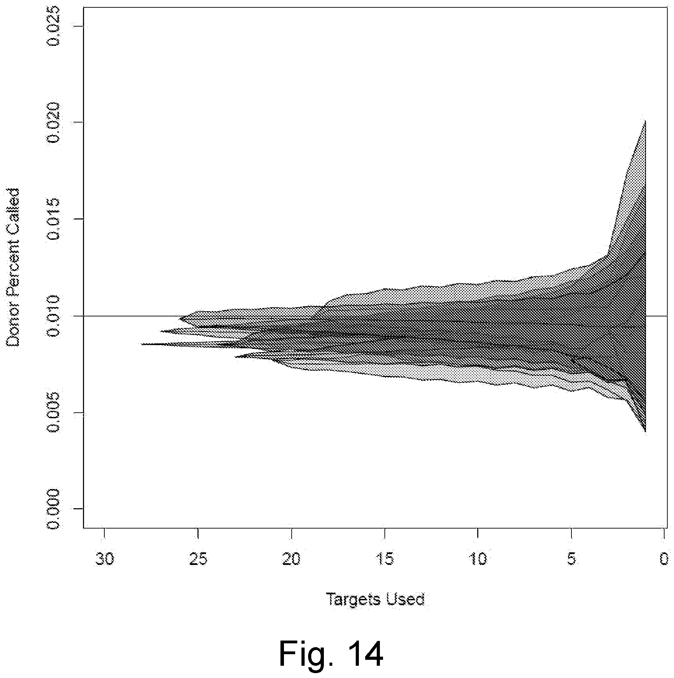

A down-sampling procedure was used, randomly censoring some targets, and then the sample was re-analyzed. The data was collected and organized by the number of informative targets in each scenario, and the outcomes were analyzed for variability. One of the seventeen runs is shown in **Fig. 13** and a composite graph of the 17 runs is present in **Fig. 14**.

As shown in **Fig. 13**, the sample had just over twenty informative targets, which gave a call of about 0.9%. As the informative targets fall, the mean does not move much, but the error bars grow. With just one informative target, the sample reported from anywhere between 0.04% and 1.13%. The data was re-calculated for coefficient of variation (CoV) and collected in **Table 1**.

**Table 1. Coefficient of Variation (data from positive control)**

| **Targets Used** | **CoV** | **Gaining One Step** |
|---|---|---|
| 1 | 41.0% | 12.6% |
| 2 | 28.5% | 5.3% |
| 3 | 23.2% | 4.1% |
| 4 | 19.1% | 1.9% |
| 5 | 17.1% | 2.7% |
| 6 | 14.5% | 0.8% |
| 7 | 13.7% | 1.7% |
| 8 | 12.0% | 0.0% |
| 9 | 12.0% | 1.5% |
| 10 | 10.5% | 0.9% |
| 11 | 9.5% | 1.2% |
| 12 | 8.3% | 0.7% |
| 13 | 7.6% | 0.8% |
| 14 | 6.8% | 0.5% |
| 15 | 6.3% | 0.7% |
| 16 | 5.6% | 0.2% |
| 17 | 5.4% | 0.9% |
| 18 | 4.5% | 0.4% |
| 19 | 4.1% | 0.6% |
| 20 | 3.6% | 0.7% |
| 21 | 2.8% | 0.3% |
| 22 | 2.5% | |

As shown in the table, when just eight informative targets are used, the sample will have a coefficient of variation about the mean of just 12%. The third column is the difference between steps in the second column, showing that a huge gain is possible between one and two informative targets, but diminishing returns are seen after about 10 informative targets, for this example.

Consequently, one can decide how much variation is tolerable and choose a data-driven target number threshold accordingly. For example, if a CoV of 5% is required, then samples below 17 informative targets could be classified as "failed-QC."

The CoV numbers and this entire analysis are predicated on the 1% donor line. A larger donor fraction would be more robust to fewer targets, and a smaller donor fraction would need more targets to have the same precision.

### Example 4 - Sensitivity and Specificity of an Exemplary MOMA Assay and Transplant Recipient Samples

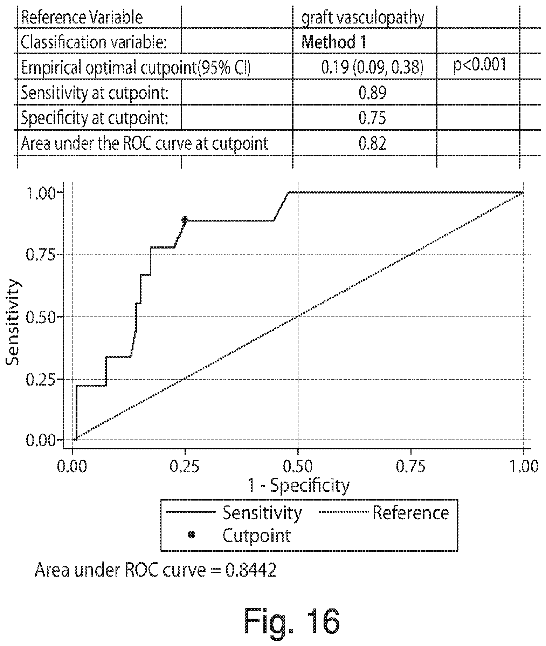

A MOMA assay was performed with a panel of 96 SNV targets on samples from 12 transplant recipients with graft vasculopathy as compared to 104 without as described above. It was found that with at least 6 informative SNV targets, the MOMA assay was able to distinguish the subjects with a sensitivity of 89% and a specificity of 75%. Results are shown in **Fig. 16**.

### Example 5 - Examples of Computer-Implemented Embodiments

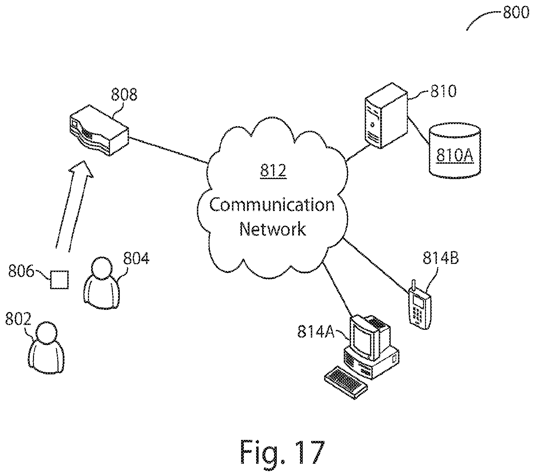

In some embodiments, the diagnostic techniques described above may be implemented via one or more computing devices executing one or more software facilities to analyze samples for a subject over time, measure nucleic acids (such as cell-free DNA) in the samples, and produce a diagnostic result based on one or more of the samples. **Fig. 17** illustrates an example of a computer system with which some embodiments may operate, though it should be appreciated that embodiments are not limited to operating with a system of the type illustrated in **Fig. 17**.

The computer system of **Fig. 17** includes a subject 802 and a clinician 804 that may obtain a sample 806 from the subject 806. As should be appreciated from the foregoing, the sample 806 may be any suitable sample of biological material for the subject 802 that may be used to measure the presence of nucleic acids (such as cell-free DNA) in the subject 802, including a blood sample. The sample 806 may be provided to an analysis device 808, which one of ordinary skill will appreciate from the foregoing will analyze the sample 808 so as to determine (including estimate) a total amount of nucleic acids (such as cell-free DNA) and an amount of a non-native nucleic acids (such as cell-free DNA) in the sample 806 and/or the subject 802. For ease of illustration, the analysis device 808 is depicted as single device, but it should be appreciated that analysis device 808 may take any suitable form and may, in some embodiments, be implemented as multiple devices. To determine the amounts of nucleic acids (such as cell-free DNA) in the sample 806 and/or subject 802, the analysis device 808 may perform any of the techniques described above, and is not limited to performing any particular analysis. The analysis device 808 may include one or more processors to execute an analysis facility implemented in software, which may drive the processor(s) to operate other hardware and receive the results of tasks performed by the other hardware to determine on overall result of the analysis, which may be the amounts of nucleic acids (such as cell-free DNA) in the sample 806 and/or the subject 802. The analysis facility may be stored in one or more computer-readable storage media, such as a memory of the device 808. In other embodiments, techniques described herein for analyzing a sample may be partially or entirely implemented in one or more special-purpose computer components such as Application Specific Integrated Circuits (ASICs), or through any other suitable form of computer component that may take the place of a software implementation.

In some embodiments, the clinician 804 may directly provide the sample 806 to the analysis device 808 and may operate the device 808 in addition to obtaining the sample 806 from the subject 802, while in other embodiments the device 808 may be located geographically remote from the clinician 804 and subject 802 and the sample 806 may need to be shipped or otherwise transferred to a location of the analysis device 808. The sample 806 may in some embodiments be provided to the analysis device 808 together with (e.g., input via any suitable interface) an identifier for the sample 806 and/or the subject 802, for a date and/or time at which the sample 806 was obtained, or other information describing or identifying the sample 806.

The analysis device 808 may in some embodiments be configured to provide a result of the analysis performed on the sample 806 to a computing device 810, which may include a data store 810A that may be implemented as a database or other suitable data store. The computing device 810 may in some embodiments be implemented as one or more servers, including as one or more physical and/or virtual machines of a distributed computing platform such as a cloud service provider. In other embodiments, the device 810 may be implemented as a desktop or laptop personal computer, a smart mobile phone, a tablet computer, a special-purpose hardware device, or other computing device.

In some embodiments, the analysis device 808 may communicate the result of its analysis to the device 810 via one or more wired and/or wireless, local and/or wide-area computer communication networks, including the Internet. The result of the analysis may be communicated using any suitable protocol and may be communicated together with the information describing or identifying the sample 806, such as an identifier for the sample 806 and/or subject 802 or a date and/or time the sample 806 was obtained.

The computing device 810 may include one or more processors to execute a diagnostic facility implemented in software, which may drive the processor(s) to perform diagnostic techniques described herein. The diagnostic facility may be stored in one or more computer-readable storage media, such as a memory of the device 810. In other embodiments, techniques described herein for analyzing a sample may be partially or entirely implemented in one or more special-purpose computer components such as Application Specific Integrated Circuits (ASICs), or through any other suitable form of computer component that may take the place of a software implementation.

The diagnostic facility may receive the result of the analysis and the information describing or identifying the sample 806 and may store that information in the data store 810A. The information may be stored in the data store 810A in association with other information for the subject 802, such as in a case that information regarding prior samples for the subject 802 was previously received and stored by the diagnostic facility. The information regarding multiple samples may be associated using a common identifier, such as an identifier for the subject 802. In some cases, the data store 810A may include information for multiple different subjects.

The diagnostic facility may also be operated to analyze results of the analysis of one or more samples 806 for a particular subject 802, identified by user input, so as to determine a diagnosis for the subject 802. The diagnosis may be a conclusion of a risk that the subject 802 has, may have, or may in the future develop a particular condition. The diagnostic facility may determine the diagnosis using any of the various examples described above, including by comparing the amounts of nucleic acids (such as cell-free DNA) determined for a particular sample 806 to one or more thresholds or by comparing a change over time in the amounts of nucleic acids (such as cell-free DNA) determined for samples 806 over time to one or more thresholds. For example, the diagnostic facility may determine a risk to the subject 802 of a condition by comparing a total amount of nucleic acids (such as cell-free DNA) for one or more samples 806 to one threshold and comparing an amount of a non-native nucleic acids (such as cell-free DNA) for the same sample(s) 806 to another threshold. Based on the comparisons to the thresholds, the diagnostic facility may produce an output indicative of a risk to the subject 802 of a condition.

As should be appreciated from the foregoing, in some embodiments, the diagnostic facility may be configured with different thresholds to which amounts of nucleic acids (such as cell-free DNA) may be compared. The different thresholds may, for example, correspond to different demographic groups (age, gender, race, economic class, presence or absence of a particular procedure/condition/other in medical history, or other demographic categories), different conditions, and/or other parameters or combinations of parameters. In such embodiments, the diagnostic facility may be configured to select thresholds against which amounts of nucleic acids (such as cell-free DNA) are to be compared, with different thresholds stored in memory of the computing device 810. The selection may thus be based on demographic information for the subject 802 in embodiments in which thresholds differ based on demographic group, and in these cases demographic information for the subject 802 may be provided to the diagnostic facility or retrieved (from another computing device, or a data store that may be the same or different from the data store 810A, or from any other suitable source) by the diagnostic facility using an identifier for the subject 802. The selection may additionally or alternatively be based on the condition for which a risk is to be determined, and the diagnostic facility may prior to determining the risk receive as input a condition and use the condition to select the thresholds on which to base the determination of risk. It should be appreciated that the diagnostic facility is not limited to selecting thresholds in any particular manner, in embodiments in which multiple thresholds are supported.

In some embodiments, the diagnostic facility may be configured to output for presentation to a user a user interface that includes a diagnosis of a risk and/or a basis for the diagnosis for a subject 802. The basis for the diagnosis may include, for example, amounts of nucleic acids (such as cell-free DNA) detected in one or more samples 806 for a subject 802. In some embodiments, user interfaces may include any of the examples of results, values, amounts, graphs, etc. discussed above. They can include results, values, amounts, etc. over time. For example, in some embodiments, a user interface may incorporate a graph similar to that shown in any one of the figures provided herein. In such a case, in some cases the graph may be annotated to indicate to a user how different regions of the graph may correspond to different diagnoses that may be produced from an analysis of data displayed in the graph. For example, thresholds against which the graphed data may be compared to determine the analysis may be imposed on the graph(s).

A user interface including a graph, particularly with the lines and/or shading, may provide a user with a far more intuitive and faster-to-review interface to determine a risk of the subject 802 based on amounts of nucleic acids (such as cell-free DNA), than may be provided through other user interfaces. It should be appreciated, however, that embodiments are not limited to being implemented with any particular user interface.

In some embodiments, the diagnostic facility may output the diagnosis or a user interface to one or more other computing devices 814 (including devices 814A, 814B) that may be operated by the subject 802 and/or a clinician, which may be the clinician 804 or another clinician. The diagnostic facility may transmit the diagnosis and/or user interface to the device 814 via the network(s) 812.

Techniques operating according to the principles described herein may be implemented in any suitable manner. Included in the discussion above are a series of flow charts showing the steps and acts of various processes that determine a risk of a condition based on an analysis of amounts of nucleic acids (such as cell-free DNA). The processing and decision blocks discussed above represent steps and acts that may be included in algorithms that carry out these various processes. Algorithms derived from these processes may be implemented as software integrated with and directing the operation of one or more single- or multi-purpose processors, may be implemented as functionally-equivalent circuits such as a Digital Signal Processing (DSP) circuit or an Application-Specific Integrated Circuit (ASIC), or may be implemented in any other suitable manner. It should be appreciated that embodiments are not limited to any particular syntax or operation of any particular circuit or of any particular programming language or type of programming language. Rather, one skilled in the art may use the description above to fabricate circuits or to implement computer software algorithms to perform the processing of a particular apparatus carrying out the types of techniques described herein. It should also be appreciated that, unless otherwise indicated herein, the particular sequence of steps and/or acts described above is merely illustrative of the algorithms that may be implemented and can be varied in implementations and embodiments of the principles described herein.

Accordingly, in some embodiments, the techniques described herein may be embodied in computer-executable instructions implemented as software, including as application software, system software, firmware, middleware, embedded code, or any other suitable type of computer code. Such computer-executable instructions may be written using any of a number of suitable programming languages and/or programming or scripting tools, and also may be compiled as executable machine language code or intermediate code that is executed on a framework or virtual machine.

When techniques described herein are embodied as computer-executable instructions, these computer-executable instructions may be implemented in any suitable manner, including as a number of functional facilities, each providing one or more operations to complete execution of algorithms operating according to these techniques. A "functional facility," however instantiated, is a structural component of a computer system that, when integrated with and executed by one or more computers, causes the one or more computers to perform a specific operational role. A functional facility may be a portion of or an entire software element. For example, a functional facility may be implemented as a function of a process, or as a discrete process, or as any other suitable unit of processing. If techniques described herein are implemented as multiple functional facilities, each functional facility may be implemented in its own way; all need not be implemented the same way. Additionally, these functional facilities may be executed in parallel and/or serially, as appropriate, and may pass information between one another using a shared memory on the computer(s) on which they are executing, using a message passing protocol, or in any other suitable way.

Generally, functional facilities include routines, programs, objects, components, data structures, etc. that perform particular tasks or implement particular abstract data types. Typically, the functionality of the functional facilities may be combined or distributed as desired in the systems in which they operate. In some implementations, one or more functional facilities carrying out techniques herein may together form a complete software package. These functional facilities may, in alternative embodiments, be adapted to interact with other, unrelated functional facilities and/or processes, to implement a software program application.

Some exemplary functional facilities have been described herein for carrying out one or more tasks. It should be appreciated, though, that the functional facilities and division of tasks described is merely illustrative of the type of functional facilities that may implement the exemplary techniques described herein, and that embodiments are not limited to being implemented in any specific number, division, or type of functional facilities. In some implementations, all functionality may be implemented in a single functional facility. It should also be appreciated that, in some implementations, some of the functional facilities described herein may be implemented together with or separately from others (i.e., as a single unit or separate units), or some of these functional facilities may not be implemented.

Computer-executable instructions implementing the techniques described herein (when implemented as one or more functional facilities or in any other manner) may, in some embodiments, be encoded on one or more computer-readable media to provide functionality to the media. Computer-readable media include magnetic media such as a hard disk drive, optical media such as a Compact Disk (CD) or a Digital Versatile Disk (DVD), a persistent or non-persistent solid-state memory (e.g., Flash memory, Magnetic RAM, etc.), or any other suitable storage media. Such a computer-readable medium may be implemented in any suitable manner, including as a portion of a computing device or as a stand-alone, separate storage medium. As used herein, "computer-readable media" (also called "computer-readable storage media") refers to tangible storage media. Tangible storage media are non-transitory and have at least one physical, structural component. In a "computer-readable medium," as used herein, at least one physical, structural component has at least one physical property that may be altered in some way during a process of creating the medium with embedded information, a process of recording information thereon, or any other process of encoding the medium with information. For example, a magnetization state of a portion of a physical structure of a computer-readable medium may be altered during a recording process.

In some, but not all, implementations in which the techniques may be embodied as computer-executable instructions, these instructions may be executed on one or more suitable computing device(s) operating in any suitable computer system, including the exemplary computer system of **Fig. 17**, or one or more computing devices (or one or more processors of one or more computing devices) may be programmed to execute the computer-executable instructions. A computing device or processor may be programmed to execute instructions when the instructions are stored in a manner accessible to the computing device or processor, such as in a data store (e.g., an on-chip cache or instruction register, a computer-readable storage medium accessible via a bus, etc.). Functional facilities comprising these computer-executable instructions may be integrated with and direct the operation of a single multi-purpose programmable digital computing device, a coordinated system of two or more multi-purpose computing device sharing processing power and jointly carrying out the techniques described herein, a single computing device or coordinated system of computing device (co-located or geographically distributed) dedicated to executing the techniques described herein, one or more Field-Programmable Gate Arrays (FPGAs) for carrying out the techniques described herein, or any other suitable system.

Embodiments have been described where the techniques are implemented in circuitry and/or computer-executable instructions. It should be appreciated that some embodiments may be in the form of a method, of which at least one example has been provided. The acts performed as part of the method may be ordered in any suitable way. Accordingly, embodiments may be constructed in which acts are performed in an order different than illustrated, which may include performing some acts simultaneously, even though shown as sequential acts in illustrative embodiments. Any one of the aforementioned, including the aforementioned devices, systems, embodiments, methods, techniques, algorithms, media, hardware, software, interfaces, processors, displays, networks, inputs, outputs or any combination thereof are provided herein in other aspects.

## Claims

1. A method of assessing an amount of donor-specific cell-free DNA in a sample from a subject, wherein the subject is a transplant recipient, the sample comprising donor-specific and subject cell-free DNA, the method comprising:
for each of 38-40 single nucleotide variant (SNV) targets, performing an amplification-based quantification assay on the sample, or portion thereof, with at least two primer pairs, wherein each primer pair comprises a forward primer and a reverse primer, wherein one of the at least two primer pairs comprises a 3' penultimate mismatch in a primer relative to one allele of the SNV target but a 3' double mismatch relative to another allele of the SNV target and specifically amplifies the one allele of the SNV target, and the another of the at least two primer pairs specifically amplifies the another allele of the SNV target,
and obtaining or providing results from the amplification-based quantification assays to determine the amount of donor-specific cell-free DNA in the sample,
wherein the donor genotype is not known, and the method further comprises predicting the donor genotype or assessing results based on a prediction of the donor genotype;
wherein the results comprise the amount of the donor-specific cell-free DNA in the sample or wherein the method further comprises determining the amount of the donor-specific cell-free DNA in the sample based on the results.

2. The method of claim 1, wherein the method further comprises obtaining the at least two primer pairs for each of the SNV targets.

3. The method of any one of the preceding claims, wherein the another primer pair of the at least two primer pairs also comprises a 3' penultimate mismatch relative to the another allele of the SNV target but a 3' double mismatch relative to the one allele of the SNV target in a primer and specifically amplifies the another allele of the SNV target.

4. The method of any one of the preceding claims, wherein:
(i) the amount is an absolute value for the donor-specific cell-free DNA in the sample, or
(ii) the amount is a relative value for the donor-specific cell-free DNA in the sample, or
(iii) the amount is the ratio or percentage of donor-specific cell-free DNA to subject cell-free DNA.

5. The method of any one of the preceding claims, wherein the amount of donor-specific cell-free DNA in the sample is less than 1% or 1.3% or 1.5%.

6. The method of any one of the preceding claims, wherein the amplification-based quantification assays are quantitative PCR assays, such as real time PCR assays or digital PCR assays.

7. The method of any one of the preceding claims, wherein the method further comprises:
(i) determining a risk in the subject based on the amount of donor-specific cell-free DNA in the sample;
(ii) selecting a treatment for the subject based on the amount of donor-specific cell-free DNA;
(iii) providing information about a treatment to the subject, or suggesting non-treatment, based on the amount of donor-specific cell-free DNA;
(iv) monitoring or suggesting the monitoring of the amount of donor-specific cell-free DNA in the subject over time;
(v) assessing the amount of donor-specific cell-free DNA in the subject at a subsequent point in time;
(vi) evaluating an effect of a treatment administered to the subject based on the amount of donor-specific cell-free DNA;
(vii) providing or obtaining the sample or a portion thereof; and/or
(viii) extracting cell-free DNA from the sample.

8. The method of any one of the preceding claims, wherein the sample comprises blood, plasma or serum.

## Patentansprüche

1. Verfahren zum Bestimmen der Menge an donorspezifischer zellfreier DNA in einer Probe von einem Subjekt, wobei das Subjekt ein Transplantatempfänger ist, die Probe umfassend donorspezifische und zellfreie Subjekt-DNA, das Verfahren umfassend:
für jedes von 38-40 Targets von Einzelnukleotidvarianten (SNV), Ausführen eines amplifikationsbasierten Quantifizierungstests an der Probe oder einem Teil davon mit mindestens zwei Primerpaaren, wobei jedes Primerpaar einen Vorwärtsprimer und einen Rückwärtsprimer umfasst, wobei eines der mindestens zwei Primerpaare eine vorletzte 3'-Fehlpaarung in einem Primer in Bezug auf ein Allel des SNV-Targets, aber eine doppelte 3'-Fehlpaarung in Bezug auf ein anderes Allel des SNV-Targets umfasst und das eine Allel des SNV-Targets spezifisch amplifiziert, und das andere der mindestens zwei Primerpaare das andere Allel des SNV-Targets spezifisch amplifiziert,
und Erlangen oder Bereitstellen von Resultaten aus den amplifikationsbasierten Quantifizierungstests, um die Menge an donorspezifischer zellfreier DNA in der Probe zu bestimmen,
wobei der Donorgenotyp nicht bekannt ist und das Verfahren ferner ein Vorhersagen des Donorgenotyps oder Bewerten von Resultaten basierend auf einer Vorhersage des Donorgenotyps umfasst;
wobei die Resultate die Menge der donorspezifischen zellfreien DNA in der Probe umfassen oder wobei das Verfahren ferner ein Bestimmen der Menge der donorspezifischen zellfreien DNA in der Probe basierend auf den Resultaten umfasst.

2. Verfahren nach Anspruch 1, wobei das Verfahren ferner ein Erlangen der mindestens zwei Primerpaare für jedes der SNV-Targets umfasst.

3. Verfahren nach einem der vorherigen Ansprüche, wobei das andere Primerpaar der mindestens zwei Primerpaare auch eine vorletzte 3'-Fehlpaarung in Bezug auf das andere Allel des SNV-Targets, aber eine doppelte 3'-Fehlpaarung in Bezug auf das eine Allel des SNV-Targets in einem Primer umfasst und das andere Allel des SNV-Targets spezifisch amplifiziert.

4. Verfahren nach einem der vorherigen Ansprüche, wobei:
(i) die Menge ein absoluter Wert für die donorspezifische zellfreie DNA in der Probe ist, oder
(ii) die Menge ein relativer Wert für die donorspezifische zellfreie DNA in der Probe ist, oder
(iii) die Menge das Verhältnis oder der Prozentsatz der donorspezifischen zellfreien DNA zu der zellfreien Subjekt-DNA ist.

5. Verfahren nach einem der vorherigen Ansprüche, wobei die Menge an donorspezifischer zellfreier DNA in der Probe weniger als 1 % oder 1,3 % oder 1,5 % ist.

6. Verfahren nach einem der vorherigen Ansprüche, wobei die amplifikationsbasierten Quantifizierungstests quantitative PCR-Tests sind, wie beispielsweise Echtzeit-PCR-Tests oder digitale PCR-Tests.

7. Verfahren nach einem der vorherigen Ansprüche, wobei das Verfahren ferner Folgendes umfasst:
(i) Bestimmen eines Risikos bei dem Subjekt basierend auf der Menge an donorspezifischer zellfreier DNA in der Probe;
(ii) Auswählen einer Behandlung für das Subjekt basierend auf der Menge an donorspezifischer zellfreier DNA;
(iii) Bereitstellen von Informationen über eine Behandlung des Subjekts oder Empfehlen einer Nicht-Behandlung basierend auf der Menge an donorspezifischer zellfreier DNA;
(iv) Überwachen oder Suggerieren des Überwachens der Menge an donorspezifischer zellfreier DNA bei dem Subjekt über die Zeit;
(v) Bewerten der Menge an donorspezifischer zellfreier DNA bei dem Subjekt zu einem späteren Zeitpunkt;
(vi) Bewerten der Wirkung einer Behandlung, die dem Subjekt verabreicht wird, basierend auf der Menge an donorspezifischer zellfreier DNA;
(vii) Bereitstellen oder Erlangen der Probe oder eines Teils davon; und/oder
(viii) Extrahieren zellfreier DNA aus der Probe.

8. Verfahren nach einem der vorherigen Ansprüche, wobei die Probe Blut, Plasma oder Serum umfasst.

## Revendications

1. Procédé d'évaluation de la quantité d'ADN acellulaire spécifique d'un donneur dans un échantillon d'un sujet, dans lequel le sujet est un receveur de greffe, l'échantillon comprenant de l'ADN acellulaire spécifique d'un donneur et du sujet, le procédé comprenant :
pour chacune des 38 à 40 variantes nucléotidiques simples (SNV) cibles, la réalisation d'un essai de quantification par amplification sur l'échantillon, ou une partie de celui-ci, avec au moins deux paires d'amorces, chaque paire d'amorce comprenant une amorce sens et une amorce antisens, l'une des deux ou plusieurs paires d'amorces comprenant un avant-dernier mésappariement 3' dans une amorce par rapport à un allèle de la SNV cible, mais un double mésappariement 3' par rapport à un autre allèle de la SNV cible et amplifie spécifiquement l'allèle de la SNV cible, et l'autre des deux ou plusieurs paires d'amorces amplifie spécifiquement l'autre allèle de la SNV cible,
et l'obtention ou la fourniture des résultats des essais de quantification par amplification pour déterminer la quantité d'ADN acellulaire spécifique du donneur dans l'échantillon,
le génotype du donneur n'étant pas connu, et le procédé comprenant en outre la prédiction du génotype du donneur ou l'évaluation des résultats sur la base d'une prédiction du génotype du donneur ;
les résultats comprenant la quantité d'ADN acellulaire spécifique du donneur dans l'échantillon ou le procédé comprenant en outre la détermination de la quantité d'ADN acellulaire spécifique du donneur dans l'échantillon sur la base des résultats.

2. Procédé de la revendication 1, le procédé comprenant en outre à obtenir les deux ou plusieurs paires d'amorces pour chacune des SNV cible.

3. Procédé de l'une quelconque des revendications précédentes, dans lequel l'autre paire d'amorces des deux ou plusieurs paires d'amorce comprend également un avant-dernier mésappariement 3' par rapport à l'autre allèle de la SNV cible mais un double mésappariement 3' par rapport à l'allèle de la SNV cible dans une amorce et amplifie spécifiquement l'autre allèle de la SNV cible.

4. Procédé de l'une quelconque des revendications précédentes, dans lequel :
(i) la quantité est une valeur absolue pour l'ADN acellulaire spécifique du donneur dans l'échantillon, ou
(ii) la quantité est une valeur relative pour l'ADN acellulaire spécifique du donneur dans l'échantillon, ou
(iii) la quantité est le rapport ou le pourcentage d'ADN acellulaire spécifique du donneur par rapport à l'ADN acellulaire du sujet.

5. Procédé de l'une quelconque des revendications précédentes, dans lequel la quantité d'ADN acellulaire spécifique du donneur dans l'échantillon est inférieure à 1 % ou 1,3 % ou 1,5 %.

6. Procédé de l'une quelconque des revendications précédentes, dans lequel les essais de quantification par amplification sont des essais de PCR quantitative, tels que des essais de PCR en temps réel ou des essais de PCR numérique.

7. Procédé de l'une quelconque des revendications précédentes, le procédé comprenant en outre :
(i) la détermination d'un risque chez le sujet sur la base de la quantité d'ADN acellulaire spécifique du donneur dans l'échantillon ;
(ii) le choix d'un traitement pour le sujet sur la base de la quantité d'ADN acellulaire spécifique du donneur ;
(iii) la fourniture d'informations sur un traitement au sujet, ou la suggestion de ne pas traiter, sur la base de la quantité d'ADN acellulaire spécifique du donneur ;
(iv) la surveillance ou la suggestion de surveiller la quantité d'ADN acellulaire spécifique du donneur chez le sujet au fil du temps ;
(v) l'évaluation de la quantité d'ADN acellulaire spécifique du donneur chez le sujet à un moment ultérieur ;
(vi) l'évaluation de l'effet d'un traitement administré au sujet sur la base de la quantité d'ADN acellulaire spécifique du donneur ;
(vii) la fourniture ou l'obtention de l'échantillon ou d'une partie de celui-ci ; et/ou
(viii) l'extraction de l'ADN acellulaire de l'échantillon.

8. Procédé de l'une quelconque des revendications précédentes, dans lequel l'échantillon comprend du sang, du plasma ou du sérum.
